# EUROPEAN PATENT SPECIFICATION

(11) **EP 3 308 758 B1**
(45) Date of publication and mention of the grant of the patent: **25.12.2019**
(21) Application number: 16193468.2
(22) Date of filing: 12.10.2016
(51) Int. Cl.: A61F 9/008

(54) **APPARATUS FOR CHANGING THE PERCEPTUAL COLOR APPEARANCE OF THE IRIS OF A HUMAN'S OR ANIMAL'S EYE**
VORRICHTUNG ZUR VERÄNDERUNG DER WAHRNEHMENDEN FARBERSCHEINUNG DER IRIS EINES MENSCHEN- ODER TIERAUGES
APPAREIL POUR CHANGER L'APPARENCE DE COULEUR PERCEPTIBLE DE L'IRIS DE L'OEIL HUMAIN OU ANIMAL

(43) Date of publication of application: 18.04.2018
(73) Proprietor: Thyzel, Reinhardt, 90542 Eckental (DE)
(72) Inventor: Thyzel, Reinhardt, 90542 Eckental (DE)
(74) Representative: Schröer, Gernot H.

(56) References cited:
- WO-A2-02/062259
- US-B2- 8 206 379

## Description

The invention is related to the field of changing the human perceptual color appearance of the iris of a human's or animal's eye.

The iris of the human or animal eye is a thin skin and muscular like tissue that surrounds the pupil and allows the pupil to be opened or closed thereby controlling the amount of light that can enter the inner eye, meaning that the level of dilation or contraction of the iris determines the amount of light that can enter the inner eye.

The pupil and iris separate the anterior eye chamber from the posterior eye chamber, both eye chambers being filled with transparent eye fluid (chamber fluid, aqueous humour) consisting mainly of water.

Behind the pupil and adjacent to the posterior chamber is the eye lens which is positioned within the lens capsule bag suspended by zonular fibres and ciliar muscles for optical accommodation of the lens. Behind the posterior chamber and the lens the vitreous body is situated, that in part is surrounded by the retina. The anterior eye chamber is closed by the cornea as a type of protective shield constituting an anterior wall for the anterior eye chamber.

The colour appearance of the iris as perceived by human beings determines the impression of the eye, and so the colour appearance of the iris is usually referred to as the "colour of the eyes". The colour of the eyes/iris can vary from blue and green to grey and brown and all kind of mixtures in between.

The "colour" of the iris as perceived by humans is generally determined by the density of pigments, i.e. melanin pigments, present on or in the anterior stroma layer of the iris. Apart from the anterior stroma layer, the iris comprises a posterior pigment layer (epithelium pigmentosum) that is located on the back side of the iris, and which is provided for light absorption preventing light from entering the inner eye through the iris.

Provided that the posterior pigmented layer is intact, i.e. contains sufficient pigments, the perceptual color is mainly determined by the density of pigments in the anterior stroma layer of the iris. The lower the density of the pigments in the anterior stroma layer is, the more the perceived eye color is shifted towards blue. Conversely, the higher the density of the pigments in the anterior stroma layer, the darker the color appearance is, ranging from green to brown, and in extreme cases almost black.

For diverse reasons, which shall not be detailed herein, and which may be induced by social and aesthetic issues, the desire of changing the color appearance of the eyes of a human being or an animal emerged. In particular, the desire of shifting the eye color towards blue emerged, and corresponding methods have been developed in recent years, wherein such methods consider reducing the amount of active melanin pigments in the anterior stroma layer.

When decreasing the density of melanin pigments in the anterior stroma layer, care has to be taken to avoid damaging the fibrovascular tissue layer of the stroma and the posterior pigment layer (epithelium pigmentosum), because such damages could cause functional impairment of the eye.

Therefore, gentle yet efficient procedures are needed for changing the melanin pigment density of the stroma layer.

Document US8,206,379 B2 describes a method for altering perceived iris color, wherein it is proposed to use a laser beam for irradiating the stroma layer such that either the melanin is destroyed, or such that the pigmented cells, i.e. the melanocytes, are selectively killed and can be removed my metabolism. Destroyed melanin or metabolized cells may be removed via the intraocular fluid over the Schlemm's Canal.

However, it has been recognized, that removal or shut off of the melanocytes or of the melanin may involve a comparatively lengthy process, meaning that the result of a treatment of the stroma layer for changing the color appearance may appear after some time, possibly necessitating further treatments of the stroma layer to finally obtain a blue color appearance of the iris, for example.

In view of this, it is an object of the invention to provide an apparatus and computer-readable non-transitory storage medium configured for changing the human perceptual color appearance of the iris of a human's or animal's eye. In particular, an apparatus and storage medium configured for changing the color appearance shall be provided that are suitable for expeditious and efficient, yet gently changing the color appearance of the iris by changing (in particular: modifying, altering) the density of melanin pigments in the anterior stroma layer of the iris.

These and further objects as described herein below are solved by the features of the independent claims. Embodiments of the invention in particular result from the dependent claims and the exemplary embodiments described in the following detailed description. Aspects, embodiments and examples of the present disclosure which do not fall under the scope of the appended claims do not form part of the claimed invention and are merely provided for illustrative purposes.

In embodiments of the invention an apparatus and a computer-readable storage medium configured for carrying out a non-surgical method for changing the human perceptual color appearance of the iris of a human's or animal's eye are provided. The method involves selectively decreasing (in particular: altering, changing, modifying) the density of pigments, specifically melanin pigments, of the anterior stroma layer, in particular the anterior border layer of the stroma layer of the iris.

The method comprises the steps of generating, by the operation of a generator module, a plurality of predefined energy quantities, and applying (in particular: impinging), by the generator module, one or more of the predefined energy quantities to the anterior stroma layer.

The one or more energy quantities may be applied to the stroma from a location positioned anterior, and/or lateral relative to the iris, for example relative to the optical axis of the eye.

The optical axis of the eye may be oriented essentially horizontally or essentially vertically during application of the energy quantities. In particular, the optical axis of the eye may be oriented vertically such that energy quantities, e.g. laser pulses, may be applied in vertical direction to the stroma, for example via an optical system positioned vertically above the eye. For example, the eye of a person may be impinged with the energy quantities in an arrangement in which the person is lying essentially horizontally on a table or similar in particular such that the optical axis of the eye is oriented essentially vertically, and in which the energy quantities are applied essentially vertically to the stroma layer. Such an arrangement may be advantageous for minimizing or reducing eye movements.

In particular, the one or more energy quantities may be applied through the anterior eye chamber 52to the stroma, meaning that the one or more energy quantities may be applied from the outside through the cornea of the eye to the stroma.

The "selective decrease" of the pigment density in this connection in particular shall mean that the energy quantity is generated and applied in such a way that substantially no damage to the cornea and fibrovascular tissue of the stroma is caused.

With the method it is provided that each of the applied energy quantities is generated and applied such that it ablates, i.e. that it is suitable for ablating (in particular: removing, avulsing), upon interacting with the anterior pigmented stroma layer, at least in part, and/or essentially completely melanocytes, i.e. melanin pigment containing cells, of the stroma, in particular of the anterior stroma layer. The energy quantities are further generated and applied in such a way that non-melanocyte tissue, of for example the stroma and the cornea of the eye, are left essentially undamaged. In particular, the one or more energy quantities in embodiments may be focused as regards the energy areal density such that, when applied through the cornea to the anterior stroma layer, the cornea, and/or also the fibrovascular structure of the anterior stroma layer, remain substantially unaffected, whilst the energy density at the stroma layer is such that the pigment density may be decreased as described in connection with the underlying invention.

The specificity for being able to ablate the melanocytes may be obtained by adequately setting or selecting the energy, power, power density, in particular surface power density, the application duration, angle of incident, focusing angle, and others for the energy quantities.

Specificity may also be obtained by generating and applying the energy quantities in pulsed form, wherein respective pulse lengths of the pulsed energy quantities may be selected so as to obtain specific ablation of melanocytes. For example, a particular pulse length (e.g. 4 ns) and pulse energy (e.g. 2 mJ) may be selected.

Further, the energy quantities at least in part are generated and applied in such a way that ablated tissue debris, and/or pigment debris of the ablated melanocytes, that is generated as an immediate (in particular: direct) cause (in particular: response) of one or more of the applied energy quantities, is, at least in part, discharged (in particular: purged) into the anterior eye chamber (in particular: the chamber between the anterior layer of the iris and the posterior endothel layer of the cornea of the eye), such that the discharged tissue/cell debris can be removed (in particular: flushed away) by a mechanically generated (in particular: artificial - in contrast to physical/natural/by medication) flow of rinsing solution through or within the anterior eye chamber.

The method of generating and applying the energy quantities so as to obtain ablation and discharge into the anterior eye chamber has the advantage, that the fibrovascular tissue of the stroma may be freed comparatively expeditious, quickly, and/or efficiently from melanocytes and melanin pigment contained therein. Thus the effects and results of applying the method to the stroma layer of the iris may become readily apparent, which may contribute to shorten the overall duration for obtaining the desired change in the color appearance of the iris.

Beyond that, generating and applying the energy quantities as proposed above, i.e. such that the ablated melanocytes are discharged into the anterior eye chamber, may also be advantageous for reducing the risk of applying further energy quantities to surface areas of the stroma that already have been sufficiently impinged with energy quantities, which may for example be the case with the known prior art methods.

Further, removing the ablated melanocyte material may avoid that incoming energy quantities, such as for example electromagnetic wave pulses, in particular laser pulses, are distorted, and/or scattered, which may contribute to enhanced specificity of ablation, whilst reducing the risk of damaging non-targeted tissue of the stroma, iris, and/or components of the eye, such as for example the retina and the like.

The apparatus is specifically adapted, configured, set up, and arranged for changing the human perceptual color appearance of the iris of a human's or animal's eye by selectively decreasing the density of pigments of the anterior stroma layer of the iris is provided. The specific adaption and configuration in particular relates to the operability, to the structure, and/or the design of the apparatus.

The proposed apparatus comprises an energy source comprising a generator module that is adapted to and set up for (in particular: programmed for) generating a plurality of predefined energy quantities of at least one of electromagnetic type and mechanical wave type.

A predefined energy quantity, as referred to herein, may for example relate to an electromagnetic wave, e.g. a light pulse, in particular laser pulse, and/or a fluid pressure wave, respectively having a predetermined energy, and preferably also a predetermined direction of propagation. A predetermined energy quantity may for example relate to an electromagnetic pulse of predefined length and energy, and/or to a fluidal pressure pulse generated within the anterior eye chamber liquid, and propagating towards and finally impinging on a predefined location (in particular: a predefined area, a predefined spot) of the anterior stroma surface/layer.

The proposed apparatus further comprises a focusing device that is adapted to and set up for focusing (in particular: directing) the generated energy quantities towards (in particular: onto) the anterior stroma layer of the iris of the eye.

The proposed apparatus may further comprise, or at least may be in mutual communication with a fluid pumping module adapted to and set up for the generation and maintenance of a predefined mechanical (in particular: artificial) flow of rinsing solution through and/or within the anterior eye chamber.

The fluid pumping module may be adapted and set up for generating a flow of 15 - 20 ml/min, and/or for generating a flow in such a way to maintain an inner eye pressure lying in the range between 16 mmHg, i.e. about 21.33 mbar, and 20 mmHg, i.e. about 26.66 mbar.

The proposed apparatus further comprises a controller unit, i.e. one or more than one controller units, that is/are programmed and set up for carrying out a method according to the invention as proposed and described in any embodiment herein. Specifically, the controller unit may be programmed and set up for carrying out the method according to the invention as described above, including any variation of the method in accordance with all the embodiments and combinations thereof as described herein.

In particular, the apparatus as proposed herein may further be implemented and set up for operating (in particular: controlling) the energy source and focusing device so as to apply (in particular: impinge) the generated predefined energy quantities, at least in part, to a predefined, in particular melanin-loaded, location (in particular: area) of the anterior layer of the stroma such that melanocyte tissue of the stroma, that is impinged with the energy quantities, is ablated (in particular: removed, avulsed) in such a way that it is discharged into the anterior eye chamber, i.e. into the fluid present in the anterior eye chamber, the discharge being a direct cause of the interaction between the energy quantities (in particular: at least one energy quantity) and the tissue.

The apparatus as proposed herein may in embodiments further be implemented and set up for operating (in particular: controlling) the fluid pumping module so as to maintain the predefined flow over a lapse of time during, and/or a lapse of time directly after applying (in particular: impinging) the energy quantities to the stroma, such that discharged melanocyte material (in particular: melanocyte debris, melanocyte material, melanin or melanin debris) at least in part is fluidly discharged from the anterior chamber.

The specific set up of the proposed apparatus in particular has the advantage, when changing (in particular: altering) the perceptual iris color of a human's or animal's eye, that melanocyte tissue, and thereby melanin pigment, can be efficiently, and within a comparatively short time of application, removed from the stroma, in particular reducing the overall time needed for changing the iris/eye color, and/or reducing the need for prolonged, and/or repeated sessions for pigment removal.

Beyond that, the maintenance of the flow of rinsing solution, which may for example comprise balanced salt solution (BSS) or ringer solution, may support ablation of melanocyte tissue by virtue of the flow of rinsing solution generated through/within the anterior eye chamber. Further advantages of the proposed apparatus in particular correspond to and result from advantages of the method as proposed herein and corresponding embodiments thereof.

In embodiments of the invention, the use of a method according to at least one of the embodiments as described herein in connection with the invention in a non-surgical treatment of the iris of a human being or an animal is proposed. The non-surgical treatment being directed to modify, in particular for aesthetic reasons, the perceived color of the iris by selectively decreasing the density of melanin pigments of the anterior stroma layer of the eye. As to advantages of the proposed use in a non-surgical treatment for changing the iris/eye color, reference is made to corresponding advantages of the underlying method and embodiments thereof as set out herein.

In embodiments of the invention, the use of an apparatus according to at least one embodiment as described herein in connection with the invention in a non-surgical treatment of the iris of a human being or an animal is proposed. The non-surgical treatment being directed to modify, in particular for aesthetic reasons, the perceived color of the iris by selectively decreasing the density of melanin pigments of the anterior stroma layer of the eye. As to advantages of the proposed use in a non-surgical treatment for changing the iris/eye color, reference is made to corresponding advantages of the underlying method and apparatus, and corresponding embodiments thereof as set out herein.

In further embodiments of the invention, a computer-readable non-transitory storage medium, or a controller-unit is proposed. The computer-readable non-transitory storage medium or controller-unit, respectively, comprise (in particular: implement) executable instructions which, when executed on (in particular: by) a computer, or when executed by operating a respective controller-unit, cause the computer or controller-unit to execute a method according to at least one embodiment as described herein in connection with the invention, and/or cause an apparatus according to at least one embodiment as described herein in connection with the invention to operate in accordance with (in particular: to carry out) a method in accordance with at least one embodiment as described herein in connection with the invention. As to advantages of the proposed computer-readable non-transitory storage medium, or the controller-unit in connection with changing the color of a human's or animal's eye, reference is made to corresponding advantages of the underlying method and apparatus, and corresponding embodiments thereof as set out herein.

The features of the embodiments as described herein, in particular herein below, are intended for being used and implemented in any of the categories referred to herein, in particular in any of the categories related to a method, an apparatus, a use, and a computer-readable storage medium or controller-unit.

In embodiments, the non-surgical method may comprise a step of providing (in particular: maintaining) a mechanically generated flow of rinsing solution through or within the anterior eye chamber and thereby removing (in particular: discharging) the tissue debris, including but not restricted to melanin pigment and corresponding cell debris, from the eye, in particular the anterior eye chamber, by means of the generated flow.

The flow may for example range between 15 ml/min and 20 ml/min, and/or the flow may be adapted and adjusted such that the inner eye pressure is kept in the range between about 16 mmHg, i.e. about 21.33 mbar, and 20 mmHg, i.e. about 26.66 mbar.

In embodiments, the step of providing the mechanically generated flow may comprise: maintaining the mechanically generated flow for a respectively predetermined lapse of time at least during, and/or after applying the predefined quantity of energy to the anterior stroma layer.

In embodiments, the mechanically generated flow may be maintained over the procedure of ablating melanocytes by applying the energy quantities. In further embodiments, the flow may be maintained as long as melanocyte debris is visible in the anterior eye chamber, wherein visibility may relate to non-magnified human perception or to 5-fold to 10-fold magnification by the use of a microscope.

In embodiments, the mechanically generated flow may optionally also be maintained during a predefined lapse of time prior to applying the predefined quantity of energy to the anterior stroma layer. Under such conditions, a predefined configuration of the flow conditions prevailing within the anterior eye chamber may be obtained, which may be advantageous for efficient tissue removal.

Removing tissue/ tissue debris by way of the maintained flow of rinsing solution, in particular by irrigation, such as for example continued irrigation, has the advantage that it is not necessary to remove all of the necrotic melanocyte tissue generated by applying the energy quantities to the stroma layer by metabolic processes from the anterior eye chamber. This in particular may be advantageous because the Schlemm's Canal, which is the natural pathway where eye humoral may exit the eye chamber, may be impaired, in particular clogged, in case that a comparatively large amount of metabolized tissue has to be precipitated via the Schlemm's Canal, as is for example the case with known methods for changing the iris color. Clogging or partial clogging of the Schlemm's Canal may lead to increased intraocular pressure, which in turn might lead to eye damages on the long term.

In embodiments, the mechanically generated flow may be maintained for at least one predetermined lapse of time in accordance with a respective, predetermined flow rate profile. The predetermined flow rate profile may be constant over time, at least for one, optionally for each, lapse of time, or may vary over time, for example in dependence of operational parameters related to the generation and application of the energy quantities.

The flow rate profile may follow a particular temporal path, wherein the flow rate profile may for example correspond to a continuous profile, and/or to a step profile.

Regarding the flow rate profile, parameters such as the energy, power, pulse rate by which the energy quantities are applied to the anterior stroma layer, and others may be used as a basis for adjusting (in particular: setting) a corresponding, in particular suitable, flow rate, i.e. a corresponding flow rate profile. By this, flow-based precipitation and ablation of the melanocyte tissue may be correlated thereby improving the overall efficiency of the proposed method.

In embodiments, at least one of a start and end point of at least one lapse of time being triggered by (in particular: synchronized with) the step of generating, and/or applying the predefined quantity of energy. The trigger may involve a lead time assigned to the start of the flow of rinsing solution, wherein the stop time may be set so as to follow-up the end of applying the energy quantities to the stroma layer.

In embodiments, the mechanically generated flow may comprise, at least during a predetermined first period of time, a laminar flow, and/or at least during a predetermined second period of time a turbulent flow. The type of flow may be selected in dependence of the applied energy or power of the energy quantities, and/or in dependence of the intended amount of melanocytes to be ablated, for example within a certain duration of time, and/or in dependence of the specific location/are of the iris that is impinged with one or more of the energy quantities.

In embodiments, the method may involve the step of partitioning (in particular: segmenting), for example by a corresponding partitioning module, at least a part of the surface area of the anterior stroma layer into a number of (in particular: a plurality of) predefined surface sections (in particular: partitions). Each of the surface sections may constitute a coherent area, and/or may have a predetermined size, wherein the surface sections may be defined in accordance with a predefined set of rules depending, for example, on the overall size/area of the iris, the pigment density, in particular the local pigment density, and others.

The surface sections may be handled in accordance with a particular sequence, wherein the sequence may for example be defined based on the actual pigment density, in particular the mean pigment density in a corresponding surface section.

Determining the surface sections, in particular carrying out a corresponding partitioning, may be based on a captured image of the iris, or of the eye. The image may for example be captured by a corresponding scanning device or module, wherein image recognition may be used to identify the iris, iris outline, iris pigment density distribution and others. The scanning device or module, may comprise a functionality for the definition of the iris or iris outline, and/or the for partitioning of the iris.

Recognition of the iris and the partitioning may be carried out automatically, at least however, semi-automatically, wherein the scanning device may comprise a functionality allowing an operator to set, define, replace, and/or correct the results of the scanning device as regards the recognition result, iris outline and the like.

After having determined (in particular: calculated) the surface sections, a number of predefined energy quantities, in particular a number of respectively predefined energy quantities, for example comprising one or more pulses/beams/bursts, may be applied to one or more of the surface sections.

The number of applied energy quantities, and other parameters of the energy quantities, such as energy, power, pulse length and the like, applied to a respective surface section may be determined (in particular: calculated) specifically for each, or at least for a certain number of the surface sections. Thus, it is possible to selectively apply the energy quantities to a corresponding surface section in accordance with respective needs, such for example resulting from the overall/mean pigment density.

In embodiments, the predefined surface sections may be processed in accordance with a predefined succession of surface sections, wherein the predefined succession may be determined by the partitioning module. The particular succession may be determined for example on the basis of iris size, local pigment density and others.

In embodiments, the predetermined surface sections, such as for example the size of one or more of the predetermined surface sections, and/or the particular succession of surface sections within the processing sequence, and/or the energy content, and/or power of the energy quantity may be determined, as has at least in part already been discussed, on the basis of (in particular: selected or set in accordance with/in dependence of) the density of pigments, and/or the specific location of the surface area on the iris, and/or the overall size of the iris, and others. In this way, it is possible to specifically adjust the operational steps for changing iris color to the specific characteristics of a particular case, thereby further increasing operational safety and prospects of success.

In embodiments, at least one parameter of the mechanically generated flow is determined (in particular: predetermined, calculated) on the basis of (in particular: in dependence on) the specific location of a respectively processed surface section, and/or the particular succession of the surface sections, and/or the density of pigments, and/or the size of a respective surface section, and/or on the basis of (in particular: in dependence on) one or more than one parameter related to generating and applying the energy quantities, such as for example, energy, power, pulse length, spot-size, surface/area energy density and others. The operational parameters of the flow may in such a way be specifically adjusted to respective particular requirements, thereby further enhancing safety and prospect of success as regards the change in iris color. In embodiments, at least one or more than one of the predefined energy quantities may be generated and applied to the stroma as at least one of:
- one or more electromagnetic waves, in particular electromagnetic wave pulses, and/or
- one or more mechanical pressure waves, in particular shock or blast waves.

The electromagnetic waves, in particular pulses, may be generated by a laser device, such as for example a pulsed laser device, in particular a Q-switched laser device, in particular Q-switched frequency doubled laser device, for example based on Nd:YAG.

The mechanical pressure waves may for example be induced (in particular: generated) by cavitation, for example plasma-induced cavitation. Further, the mechanical pressure waves may be directly induced by a plasma blast, and/or by an electromagnetic wave pulse, generated within liquid contained in, or liquid that is in direct fluid contact with the anterior eye chamber, specifically a fluid contained in the anterior eye chamber. In embodiments, one or more plasma blasts, at least on part, may be induced by an interaction of electromagnetic wave pulses with the stroma layer, in particular with the fibervascular structure of the stroma.

In embodiments, the method may comprise a step of determining the local pigment density, and/or the thickness of melanocyte layer at the anterior stroma layer, wherein it may further be provided that generating, and/or locally applying one or more of the energy quantities, in particular pressure pulses and/or laser pulses, is carried out in dependence on (in particular: on the basis of) a respective local density of pigments, and/or local thickness of the anterior melanocyte layer/density.

In particular with such embodiments, for example in which laser pulses of a Q-switched laser device are applied to the iris for color change, enhanced operational safety as regards potential iris impairments may be obtained. Further, the process of changing the iris color may be adapted to the particular needs of the case, which may help to reduce the overall time needed for changing the color in accordance with the aimed result.

In embodiments, the at least one or more of the energy quantities may be generated as electromagnetic wave pulses directly applied to the iris tissue by the operation of a laser device operating in a wavelength range between 488 nm to 580 nm, or in a wavelength range between 522 nm to 541 nm, or with a wavelength corresponding substantially to 532nm, or in a wavelength range between 976 nm to 1160 nm, or in a wavelength range between 1044 to 1082 nm, or with a wavelength corresponding substantially to 1064 nm.

In embodiments, the at least one or more of the energy quantities may be generated as electromagnetic wave pulses having a pulse frequency in the range between 3 Hz and 300 Hz, and/or having a pulse length lying in the range between 2 ns and 6 ns, or corresponding substantially to 4 ns.

In embodiments, an optical system of the laser device may be operated or adjusted to generate an, in particular pulsed, laser beam for generating at least one or more than one, in particular substantially all, of the energy quantities (14), the laser beam having a focusing angle lying in the range between 10 degrees and 18 degrees, optionally between 13 degrees and 16 degrees, further optionally lying substantially at about 14 degrees.

In embodiments, the energy quantities may be guided from a light emitting element to the optical system via a fiber optical system. The fiber optical system may comprise an optical fiber which may have a fiber-optic core diameter lying in the range between 270 µm and 290 µm, in particular at about 280 µm.

In embodiments, one or more than one of the energy quantities, in particular all energy quantities, may be applied to the anterior stroma layer substantially in vertical direction. In other words, the optical axis of the eye may be oriented vertically, and the energy quantities may be applied essentially according to the orientation of the optical axis of the eye. By using a vertical axis (in particular: direction) for applying the energy quantities, the person whose eye color shall be changed may be positioned horizontally, which may lead to reduced head and/or eye movement.

The parameters mentioned above in particular have been proven to be particularly suitable for ablating melanocytes from the anterior stroma layer so as to discharge (in particular: purge) corresponding tissue and pigment into the liquid contained in the anterior eye chamber such that corresponding discharged tissue and pigment material can be removed via the maintained flow of rinsing fluid.

In embodiments, the at least one or more of the energy quantities may be generated as mechanic wave pulses as a direct cause of a plasma-induced burst pulse. The burst pulse may for example be generated by direct interaction of an electromagnetic wave pulse, such as a laser pulse with a laser target, such for example a solid state material, e.g. titanium, that is in fluid communication with the intraocular humor.. A plasma-induced burst may also be generated by the interaction of an electromagnetic wave pulse with the anterior stroma layer, in particular the fibrovascular structure of the stroma layer.

Electromagnetic wave pulses for generating blast pulses may for example have a wavelength of 532nm or 1064 nm, and may have an energy of about 2 mJ per pulse.

In embodiments, the plasma-induced burst pulse may be generated by direct interaction of a laser pulse with rinsing-solution within the anterior eye chamber and/or, as already mentioned, with the fibrovascular structure of the anterior stroma layer.

For example, a plasma-induced burst pulse may be generated within the fluid contained in the anterior eye chamber near the surface area intended for being impinged with the burst pulse, wherein a laser pulse may be applied to the liquid and/or stroma layer (in particular: stroma fibrovascular structure) such that direct cavitation/plasma-induced burst pulses may be generated.
In embodiments, generating the energy quantities may involve operating or adjusting an optical system of the laser device such that a pulsed laser beam for generating at least one or more than one, in particular substantially all, of the energy quantities is generated, wherein the pulsed laser beam comprises a focusing angle lying in the range between 10 degrees and 18 degrees, optionally between 13 degrees and 16 degrees, further optionally lying substantially at about 14 degrees. In other words, the method may involve the use of an optical focusing device leading to a focusing angle lying in the given range or ranges. Using such a focusing angle in particular has the advantage that, when applying the energy quantities, in particular laser pulses, from the outside of the eye, through the cornea, that the course and increase of the energy density, in particular the area energy density, of the laser beam towards the target on or at the anterior stroma layer is such that the cornea remains substantially unaffected by energy quantities passing therethrough, whilst melanocytes at or on the anterior stroma layer may be ablated leaving the fibrovascular stroma structure essentially unaffected.

In embodiments, the energy quantities may be guided from a source towards the intended target, at least in part, via a fiber optical system. For example, the energy quantities, e.g. laser pulses, may be guided from a corresponding light emitting element to an optical system implemented and provided for applying, in particular focusing, the energy quantity to the anterior stroma layer via a fiber optical system. Using such a fiber optical system including for example a flexible optical fiber, has the advantage that the optical axis of the eye to be impinged with the energy quantities may be freely orientated. For example, the optical axis of the eye may, according to respective preferences, be oriented vertically or horizontally, or according to any other direction respectively suitable for ablating melanocyte pigment from the anterior stroma layer.

A corresponding fiber optical system of that can be used when performing the method may comprise an optical fiber having a fiber-optic core diameter lying in the range between 270 µm and 290 µm, in particular at about 280 µm.

In embodiments, the method may further involve tracking, by a tracking module, e.g. an optical tracking module, a position (in particular: location), and/or shape, and/or movement of the eye or one or more of the components of the eye, for example the iris or the pupil, e.g. relative to a, in particular fixed, spatial reference point. In such embodiments, each of the generated, predefined energy quantities may be applied to the stroma layer in dependence on (in particular: conditional on) the tracking result. The tracking result, i.e. the outcome of the tracking procedure, may for example comprise a determination indicating whether or not the location, and/or size of the iris or pupil has changed, and/or whether or not the eye/iris has been moved.

In embodiments, in which such tracking is involved, the application (in particular: the release) of an energy quantity may be inhibited (in particular: blocked) in case of a positive determination of a position, size, and/or shape change, and/or in case of a positive determination of a movement.

Tracking in particular has the advantage that the iris or other parts of the eye, such as for example the retina, may be impairment or even damaged by for example falsely applying an energy quant due to eye movement going along with iris movement. Adequately operating the tracking module in particular may contribute to overall safety, in particular in cases where trained medical staff conducts the method of changing eye color. Further tracking is suitable and helpful for automating or at least semi-automating the proposed method for changing eye color.

In embodiments, the tracking result may not only be used for inhibiting the application (in particular: the release) of one or more of the energy quantities. The tracking result, which may in embodiments involve a continuous tracking of the eye or a corresponding, suitable component of the eye, e.g. the iris or the pupil, may also and/or in the alternative be used for target control. In particular, the target setting of the energy quantity may, for target control, be relocated in accordance with one or more of a change in position, a change in location, a change in shape, and a movement.

Target control in particular shall mean that the tracking result may be used for controlling for example the generator module, or other suitable module, such that the released energy quantity is applied to the correct (in particular: intended) target point (in particular: target area) of the anterior stroma layer. By this, the target setting for the one or more energy quantities may be moved (in particular: adjusted) in accordance with (in particular: synchronously with) a detected eye movement, and/or a detected change in position, location, and/or shape.

The eye tracking-based target setting control may be implemented such that inhibition of applying the energy quantity and/or relocation of the target point/area are performed (only) in instances in which the target setting lies outside of the stroma area intended for being treated for color change purposes, and/or in case that the target setting differs from the actual target point/area by more than a predetermined threshold.

Providing such tracking options and controls, in particular by adequately synchronising target setting with eye movement and/or with corresponding changes in or of the eye/iris, may improve execution of the method, and may contribute to enhanced safety in particular with regard to possible damages to non-melanocyte tissue.

For tracking the eye and/or iris movement in a method or system for changing the eye color as described herein, an eye tracker using a stereoscopic camera system, in particular in connection with infrared light, may be used. The eye tracker may be configured to operate independently from ambient light, or may require specific illumination. The exe tracker, in particular the eye tracking, may involve determining 3D-position of pupil or iris, 3D-viewing direction, pupil size, viewing focus relative to a predefined surface/object.

Target control may in embodiments involve applying the energy quantity, at a particular target point or target area of the anterior stroma layer. The particular target point may be moved between a plurality of target points according to particular pattern. The particular pattern may involve repeatedly selecting or determining one or more than one target point from a plurality of target points. A selected or determined target point may have, in a direction substantially parallel to the anterior stroma layer of the iris, a predefined minimum distance from a previous target point, wherein a first or initial target point may be pre-set or selected randomly, for example. The predefined minimum distance may correspond to the diameter of the pupil measured when carrying out the method, wherein, with respect to a plane running parallel to and comprising the optical axis of the eye, each target point may optionally be positioned on an opposite side of such a plane as regards a respective previous target point, wherein the rotary position of such a plane relative to the optical axis may vary between subsequent target points.

In embodiments, the succession of target points is selected/determined such that the local energy, in particular heat input to the anterior stroma layer and/or cornea, caused by applying two subsequent energy quantities, in particular laser pulses, resides below a pre-defined value.

In embodiments, the method may involve coupling, prior to applying one or more than one of the energy quantities, an eye shielding element to the eye, in particular to the outer cornea layer. In other words, the method may involve the use of an eye shielding element. The eye shielding element is implemented and may be positioned on the outer cornea layer of the cornea such that energy quantities impinged to the eye are prevented from entering the posterior eye chamber and/or from impinging the retina of the eye. The use of such an eye shielding element has the advantage that the retina can effectively be shielded from energy quants applied to the eye.

The eye shielding element may in embodiments of the invention, that may be claimed as independent claims as well, be implemented in or as a composite structure, wherein the composite structure may comprise an absorption layer for absorbing energy quantities impinging thereon, and an adhesion layer for coupling with the outer cornea layer.

The absorption layer may in embodiments comprise one or more than one metallic layers. At least one or more than one of the one or more metallic layers may be made from stainless steel, i.e. may be implemented as a stainless steel layer.

The coupling between the adhesion layer and the outer cornea layer, which is in general covered by a liquid film, may be based on adhesive forces between the adhesion layer and the cornea layer and/or cornea liquid film and/or cohesion-induced adhesion of the adhesion layer. Adhesive attachment to the outer cornea layer may be accomplished by an open-pored material layer, in particular foamed layer, preferably comprising and/or made from a polyvinyl alcohol material. Thus, in embodiments, the eye shielding element may comprise an open-pored material layer, in particular foamed layer, preferably comprising and/or made from a polyvinyl alcohol material, that is for example attached to the shielding layer.

The eye shielding element may partly, in particular in the adhesion layer when present, be filled with or absorb natural liquid from the outer side of the cornea thereby adhering to the cornea like for instance a paper tissue or blotting paper would.

The eye shielding element may be placed to cover the pupil of the eye, which, whilst applying the energy quantities, may have a diameter lying in the range between 1 mm and 1.5 mm, in particular at about 1 mm.

The eye shielding element in embodiments, may have a convex, and/or circular shape, and/or may have a diameter lying in the range between 0.5 mm and 3 mm, or in the range between 1 mm and 2 mm, or substantially at about 1.5 mm.

The eye shielding element may be used for covering the pupil in case that the eye is exposed to laser radiation so as to avoid, by covering the pupil, laser radiation entering the posterior eye chamber, and possibly the retina.

In case that an eye shielding element is used, the method may involve verifying the correct position of the eye shielding element relative to the pupil of the eye, which may be executed by a scanning module, for example.

In embodiments, the method may comprise a step of scanning, by a/the scanning module, at least the iris or sections thereof, and/or the anterior eye chamber at least one of prior to, during, and after application of the energy quantities. The scanning result may comprise a representation of the eye chamber(s) and/or the iris, in particular the anterior stroma layer. The scanning result may be transformed into a parameterized model of the eye/iris/anterior stroma layer, wherein the scanning result, in particular the parameterized model, may be used for target determination, and may be used for (in particular: as the basis of) target tracking, in case that a tracking module is present and used.

The scanning result may be used to determine a shape of the iris and/or a track, pathway and/or succession of target points to be impinged with the energy quantities based on the scanning result. In particular, the scanning result may be used at least in part for target point determination, which may be carried out prior to melanocyte ablation and/or throughout carrying out the method.

The scanning result may in embodiments be stored, for example after a predetermined number of applied energy quantities, e.g. after finalizing method and/or after certain phases, in a non-volantile memory. Such scanning results may be used as documentary evidence for the course of events and/or as an operational history for the procedure of changing the eye/iris color.

In embodiments, the scanning result of the scanning module may be utilized for target setting, in particular for determining (in particular: calculating) a location of impingement or an averaged location of impingement respectively indicating an actual location on the anterior stroma layer or the iris where one or more of the energy quantities indeed impinged on the anterior stroma layer. When using such a functionality, the target locations of impingement may be tracked, and the tracking result may be stored in a database, for example together with operational parameters related to the generation and application of the energy quantity or quantities.

In embodiments, the flow of rinsing solution within or through the anterior eye chamber may be controlled on the basis of the scanning result.

In embodiments, the density of pigments, in particular the local density of pigments such as for example a pigment profile/distribution, at least, however, a parameter representative of the density/local density of pigments may be determined (in particular: calculated) based on the scanning result.

The determined density of pigments, in particular local density of pigments, the pigment profile and/or the pigment distribution may be used for controlling the generation and/or application of one or more of the energy quantities to the anterior stroma layer. In particular, the determined density of pigments, in particular local density of pigments, the pigment profile and/or the pigment distribution may be used as a parameter for determining (in particular: setting) specificities of the predetermined energy quantities, such for example specificities (in particular: parameters) of predetermined laser pulses and/or pressure pulses.

For example, energy, pulselength, irradiance, and other parameters of for example of a corresponding light/pressure pulse, may be set at least on part on the basis of the density of pigments, the pigment profile, and/or distribution. Scanning a corresponding section of the eye/iris may be conducted immediately prior to applying a corresponding energy quantity and/or during and/or after applying energy quantities to the anterior stroma layer of the iris.

In embodiments, it may be provided, that, based on the scanning result, a change, in particular local change, in the density of pigments, at least, however, a parameter representative of a change/local change in the density of pigments of the anterior stroma layer may be determined (in particular: calculated). Based on such determination the generation and/or application of one or more of the energy quantities may be controlled based on the determined change/local change in the density of pigments, or a respective parameter representative of a change/local change in the density of pigments.

In embodiments, the scanning result may be used as a basis for generating and providing for display on a display screen one or more than one display objects to an operator/supervisor executing of the method. Such display objects in particular may comprise one or more of: the iris, sections of the iris, iris color, iris color change, pigment density, pigment density and others.

In particular in such embodiments, it may be provided that further operational parameters related to the execution of the underlying method may be provided for display on a/the display screen. For example, one or more operational parameters related to the generated/applied energy quantities, for example their energy, wavelength, etc., related to the points of impact of one or more of the applied energy quantities on the anterior stroma layer, in particular one or more of a one or more past and future points of impact may be provided for display. Further, a first indication representative of a change, in particular local change, of the density of pigments, and a second indication representative of processed, and/or unprocessed surface areas of the anterior surface of the stroma layer, may be provided for display on a display screen for the operating/supervising user.

In embodiments, an apparatus as proposed herein and set up for carrying out the proposed method of changing the color of the iris, i.e. the color of the eye, may comprise a microscope module, for example implemented as or comprising a stereoscopic microscope.

The microscope module may be arranged and positionable in such a way that an operator/supervisor operating the apparatus in accordance with the proposed method is able to observe at least the anterior surface of the stroma layer of the eye. The microscope module may be implemented to allow imaging of at least the iris, and may optionally be implemented (in particular: set up) to provide for display the imaged section of the eye.

In embodiments, the apparatus may further comprise a slit lamp module comprising a slit lamp and corresponding optical elements allowing the operator/supervisor of the method to throw a sheet of light into the eye/onto the anterior stroma layer. The slit lamp and microscope module may be adapted for (in particular: set up for) mutual interaction thereby implementing a slit lamp microscope for investigating at least the anterior stroma layer of the iris, in particular whilst carrying out the proposed method.

In embodiments, the apparatus may comprise an eye shielding element implemented and set up for being coupled to the eye, in particular the outer cornea layer in such a way to prevent energy quantities from entering the posterior eye chamber and/or from impinging the retina of the eye.

The eye shielding element in embodiments may have a composite structure comprising an absorption layer implemented for absorbing energy quantities impinging thereon for the purpose of changing the color of the eye. This means, that the absorption layer is configured such that energy quantities used for ablating melanocyte pigment of the anterior stroma layer may be effectively, in particular essentially completely, be absorbed.

In embodiments, the eye shielding element may comprise, in addition to the absorption layer, an adhesion layer for coupling with the outer cornea layer, in particular by adhesive and/or cohesive forces, generated for example between the adhesion layer and the cornea, the adhesion layer and lacrimal fluid, and/or by lacrimal fluid covering the cornea. The adhesion layer may be attached to one side of the absorption layer.

In embodiments, the absorption layer may comprise one or more than one metallic layers, in particular stainless steel layers. The metallic layers may be selected such that energy quantities needed for ablating melanocyte pigments from the anterior stroma layer are absorbed, i.e. that at least such energy quantities are prevented from passing the eye shielding element.

The adhesion layer may in embodiments comprise an open-pored material layer, in particular foamed layer, preferably comprising and/or made from a polyvinyl alcohol material, which may be flexible to some extent so as to adapt to the outer shape of the cornea. The adhesion layer may provide the advantage that slipping away from the intended position due to the natural liquid layer at the outer side of the cornea may be prevented.

The eye shielding element may partly, in particular in the adhesion layer when present, be filled with or absorb natural liquid from the outer side of the cornea thereby adhering to the cornea like for instance a paper tissue or blotting paper would.

The eye shielding element may in embodiments have a convex, and/or circular shape, and/or may have a diameter lying in the range between 0.5 mm and 3 mm, or in the range between 1 mm and 2 mm, or substantially at about 1.5 mm.

In embodiments, the energy source, which is implemented for generating the energy quantities, may comprise as a generator module at least one (in particular: one or more than one) light emitting element. The light emitting element may comprise or be implemented as a laser device, for example as a Q-switched laser device, in particular a Q-switched frequency doubled laser device. The laser device may for example be of the type of a Nd:YAG laser device.

The laser device may be operable (in particular: set up for being operated) so as to generate at least some of the predetermined energy quantities, comprising for example one or more than one laser pluses. The generated energy quantities may be generated for emission in the form of light pulses. Further, the laser device may be operable, for example by means of a corresponding focusing module, to direct the light/laser pulses towards and onto the anterior stroma layer.

For example, the focusing module may be configured for (in particular: set up for) directing one or more generated light/laser pulses to a corresponding specific target point (in particular: target location) of impingement. As already mentioned, the target point of impingement may, at least in part, determined based on a scanning result of the anterior layer of the iris.

Adequately emitting the one or more light pulses towards and onto the anterior layer results in melanocyte ablation, i.e. in ablation of melanocyte cells located on the anterior stroma layer, which, as has been explained in detail above, leads to a change in the density of pigments in/on the anterior stroma layer, which in turn results in a change of the perceptual color of the iris/eye.

The light emitting element may be operable to generate and emit light, in particular light pulses, having a wavelength ranging between 488 nm and 580 nm, such as for about example 532 nm, or having a wavelength ranging between 976 nm to 1160 nm, or between 1044 to 1082 nm, such for example about 1064 nm. The light emitting element may further be operable to generate light pulses, in particular laser pulses, having a duration in the range between 2 ns and 6 ns, in particular about 4 ns. The light pulses may for example have an energy of about 2 mJ. Further, the light pulses may be generated with a pulse frequency laying in the range between 3 Hz and 300 Hz.

The light emitting element may be implemented to be able to generate light pulses having a peak power ranging between 0.1 MW to 0.5 MW.

Further, the light emitting element may be implemented to generate a laser spot having a diameter ranging between 200 µm and 600 µm, for example ranging about 400 µm.

In particular, the mentioned parameters have turned out to be suitable for ablating melanocytes of the anterior stroma layer so as to be discharged into the anterior eye chamber, in particular into the fluid contained in the anterior eye chamber.

In embodiments, the energy source may comprise an optical system implemented and set up for generating, during ablating the melanocyte pigments, a laser bean, in particular pulsed laser beam, having a focusing angle lying in the range between 10 degrees and 18 degrees, optionally between 13 degrees and 16 degrees, further optionally lying substantially at about 14 degrees. Such focusing angles may be effective in leaving the cornea of the eye essentially unaffected when applying the laser pulses to the anterior stroma layer, as has been explained in detail already above.

The optical system may in embodiments be implemented and set up for applying the energy quantities to the anterior stroma layer substantially in vertical direction. As already mentioned, the optical system may also be implemented to apply the energy quantities in horizontal direction or any suitable direction therebetween.

In embodiments, the apparatus, in particular the optical system, may comprise a fiber optical system implemented and set up for guiding energy quantities generated by the light emitting element towards the target location (in particular: site of application).

The fiber optical system may in embodiments comprise an optical fiber having, for example, a fiber-optic core diameter lying in the range between 270 µm and 290 µm, in particular at about 280 µm.

In embodiments, the fiber optical system may be coupled between the light emitting element and the optical system such that the energy quantities intended for ablating melanocyte pigments from the anterior stroma layer may be guided from the light emitting element located at a remote location to the optical system located near (in particular: closer to) the eye. Using such a fiber optical system may provide improved flexibility and adaptability to respective needs, in particular as regards the orientation of the optical axis of the eye while changing the color of the eye.

In embodiments, the energy source may comprise as a generator module a pressure wave generator, wherein the pressure wave generator may be implemented for (in particular: set up for) being operated to generate at least some of the predefined energy quantities, and to emit the generated energy quantities in the form of mechanical pressure waves (in particular: shock waves, blast waves) towards and onto the anterior stroma layer so as to change (in particular: alter, modify) the density of pigments of the anterior stroma layer by ablating melanocyte cells and discharging them into the anterior eye chamber.

Light pulses and pressure waves may either be used alone for changing the pigment density, or, in embodiments, both light pulses and pressure waves may be used concurrently or consecutively for changing the density of pigments, in particular in obtaining the intended pigment density change, i.e. iris color.

The pressure wave generator may in embodiments be operable (in particular: implemented or set up) to generate the pressure waves by induced cavitation within a liquid that at least is in liquid contact with the eye liquid as contained in the anterior eye chamber.

As has been shown, the underlying invention and embodiments thereof are suitable for changing the human perceptual color appearance of the iris of a human's or animal's eye in an expedient, gently, and efficient way.

Exemplary embodiments of the invention will now be described in connection with the annexed figures, in which:
- FIG. 1: shows a schematic representation of a human eye together with schematic representations for carrying out the underlying method;
- FIG. 2: shows an enlarged view of a section of FIG. 1, 2 including the iris of the eye;
- FIG. 3: shows a further schematic view of the eye and a schematic embodiment of an apparatus for carrying out the underlying invention;
- FIG. 4: shows a further representation of the human eye together with further details for applying laser pulses in connection with the underlying method;
- FIG. 5: shows a schematic view of the eye and further details of an apparatus for carrying out the underlying invention;
- FIG. 6: shows the enlarged view of FIG. 3 together with an eye shielding element;

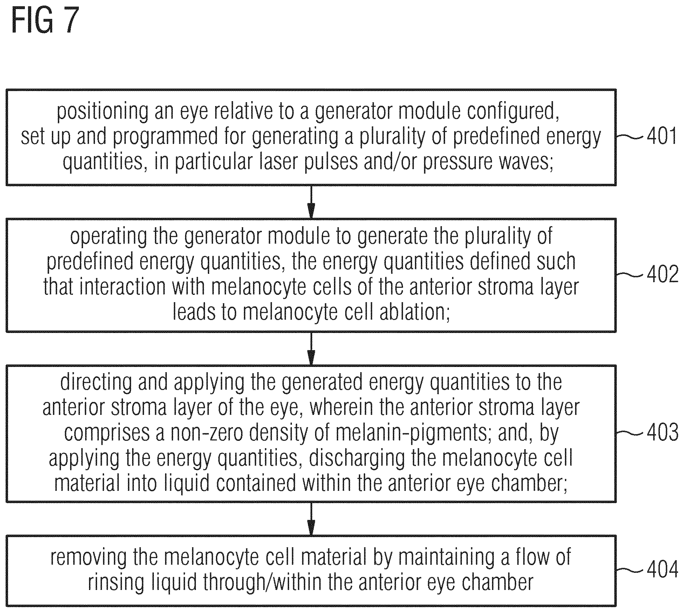

- FIG. 7: shows an example flow chart for carrying out a method according to the invention.

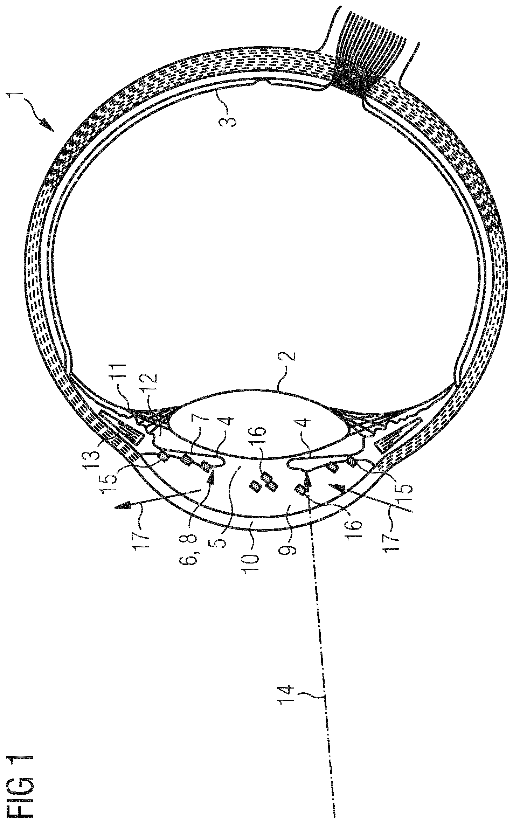

In FIG. 1, a schematic representation of a human eye 1 is presented together with elements or components of an apparatus for carrying out the underlying method.

As regards the human eye 1, components thereof are described in such an extent which is considered adequate for fully understanding the underlying method.

The human eye 1 represents an optical system with which real-life objects are imaged via a lens 2 onto the retina 3 of the eye 1. Light incidence into the eye 1, and therefore the amount of light incident on the retina 3, is controlled by a variable aperture represented by a comparatively thin, circular structure in the eye 1, known as the iris 4.

By a muscular system, the eye 1 is able to vary the diameter and size of the central iris opening, i.e. the pupil 5 and thus the amount of light reaching the retina 3.

The iris 4 consists essentially of two layers, the so-called stroma layer or stroma 6, which is located at the front (i.e. anterior) of the iris 4 oriented towards the outside of the eye 1, and a layer of pigmented epithelial cells 7 positioned on the back side (i.e. posterior) of the iris 4. The pigmented epithelial cells have the function to absorb incoming light to such an extent that the iris indeed functions as an aperture, defining the diameter of the pupil 5, for the optical system of the eye 1.

The pigmented epithelial cells comprise melanin as the pigment, which is an efficient light absorber, in particular as regards light in the visible spectrum. The anterior stroma layer 8, i.e. the layer of the stroma oriented towards the outside of the eye, i.e. towards the anterior eye chamber 9, may comprise a certain amount of melanocyte cells containing melanin pigment. The amount or density of melanin pigment in the anterior stroma layer 8 is not fixed, and may vary from individuum to individuum.

Just for the sake of completeness, the anterior eye chamber 9 is delimited to the outside by the cornea 10. The anterior eye chamber 9 defined and located between the iris 4 and the cornea 10 is filled with aqueous humor, wherein aqueous humor is formed in the ciliar body 11 of the eye 1 where it is secreted into the posterior eye chamber 12. From the posterior eye chamber 12 the aqueous humor passes through between the iris 4 and lens 2 into the into the anterior eye chamber 9, from where it is discharged over the Schlemm's Canal 13 of the eye 1.

The melanin pigments comprised by the melanocytes alter the absorption and reflection behaviour of the iris 4, more specifically of the stroma 6. The melanin pigments, in particular the density of melanin pigments in or of the anterior stroma layer 8 greatly influence the perceived eye color, which is used as a synonym for the perceived color of the iris.

As a matter of fact, if no or hardly any melanin is present in or on the anterior stroma layer 8, the perceived eye color is blue, whereas with increasing melanin, or melanin-density, the perceived eye color shifts towards green, hazel, and brown. The melanin-density occurring naturally in the anterior stroma layer 8 is determined by the phenotype of the individuum under consideration.

For some reasons, inter alia driven by aesthetic and/or social forces, there exists the desire to change perceptual eye/iris color from brown, green etc. to blue. Thus, corresponding methods have been developed, for changing the perceptual eye/iris color by removing melanin pigments of the anterior stroma layer of the eye. For example in US 8,206,379 B2, a laser-based approach is described in which melanocyte cells of the anterior stroma layer, containing melanin pigment, are killed, such that the melanocytes and corresponding melanin pigments can be removed by macrophage digestion, and removed from the eye chamber via the so-called Schlemm's Canal depicted in FIG. 1 for convenience with reference sign 13.

As already discussed, macrophage-based removal of the pigmentation of the anterior stroma layer 8 may adversely affect the operability of the Schlemm's Canal 13, which may lead to increased intraocular pressure, which in turn may impair ocular health.

The method according to the underlying invention is based on a different approach, which has been found by elaborate investigation of the anterior stroma layer 8 and the pigmentation of the anterior stroma layer 8.

Specifically, it has been found, that the melanin-containg melanocyte cells of the anterior stroma layer 8 can be ablated by specifically impinging the anterior stroma layer with energy quantities, of for example electromagnetic wave pulses and/or fluidal pressure pulses, or similar, such that the melanocyte cells and corresponding cell debris and pigment is discharged into the aqueous humor of the anterior eye chamber 9 as a direct cause of the applied energy quantity, such that the ablated material can be removed via a mechanically generated flow of rinsing solution through or within the anterior eye chamber 9.

This process underlying the present invention is schematically indicated and depicted in FIG. 1, showing inter alia applying an energy quantity, by for example one or more laser pulses 14 to the anterior stroma layer 8 such that melanocyte cells 15 bound to the stroma 6 are, as a direct cause of the impinged one or more laser pulses 14, discharged into the aqueous humor/liquid contained in the anterior eye chamber 9. Discharged melanocyte material is depicted with reference sign 16 in FIG. 1.

The discharged melanocyte material 16 can then be removed from the anterior eye chamber 9, for example, by maintaining a mechanically generated flow of rinsing solution through the anterior eye chamber, which flow is indicated by two arrows 17 in FIG. 1. The flow of rinsing solution 17 may for example be generated by an irrigation device (not explicitly shown in FIG. 1), allowing the generation and maintenance of a, for example, continuous flow of rinsing fluid through the anterior eye chamber 9.

Maintenance of the flow of rinsing solution and applying the laser pulses 14 in order to ablate the melanocytes and discharge them directly into the anterior eye chamber 9 may, for example, be executed by trained medical staff, not requiring the intervention of a medical professional.

By removing the melanocytes of the anterior stroma layer 8, the perceptual color of the eye 1 may be changed from a brown/green shade or color towards blue. Further, by conducting the method such that the melanocyte material is directly discharged into the anterior eye chamber 9, the melanocyte material can be removed by maintaining the flow of rinsing solution 17, thereby avoiding impairments of the naturally occurring exhaust processes for the aqueous humor over the Schlemm's Canal 13.

Beyond that, by directly removing the melanocytes, the pigment density of the anterior stroma layer 8 may be changed instantaneously, meaning that the result of pigment removal may be directly checked or monitored, which may lead to more efficient, and overall shortened procedures for changing the perceptual eye color to a respectively desired color.

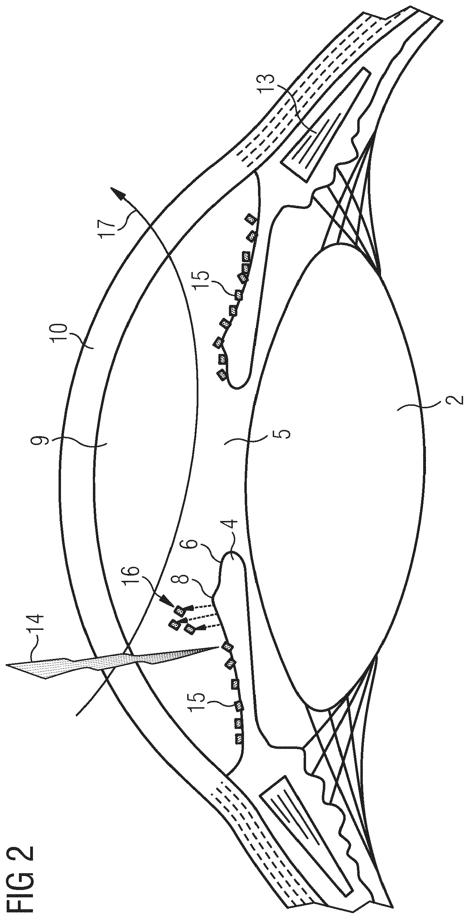

FIG. 2 shows an enlarged view of the iris 4 and stroma 6 region of the eye as shown in FIG. 1. Specifically, FIG. 2 shows one or more laser pulses 14 applied to and impinging on the anterior stroma layer 8. As a direct consequence of applying the one or more laser pulses 14, melanocyte cells 15, which under normal conditions are bound to the anterior stroma layer 8, are, by the direct action of the one or more laser pulse 14, directly discharged into the aqueous humor/liquid/aqueous humor rinsing solution mixture that is present in the anterior eye chamber 9. The discharged melanocyte cells and melanocyte material 16 may be flushed away and out of the anterior eye chamber 9 by the mechanically generated flow 17 of rinsing solution generated through/across the anterior eye chamber 9.

In order to obtain adequate and/or improved removal of the discharged melanocytes 16, it is possible to maintain the mechanically generated flow 17 of rinsing solution for a predetermined lapse of time during, prior to, and/or after applying the predefined energy quantities, e.g. the one or more laser pulses 14.

The flow of rinsing solution 17 may be provided according to a predetermined profile, in particular flow rate profile, which may be constant over time, or which may vary according to a predefined time course. Variations of the profile may for example correspond or synchronized with the pulse rate and/or with the density of pigments of a respective area of the stroma 6 where the energy quantities are respectively applied.

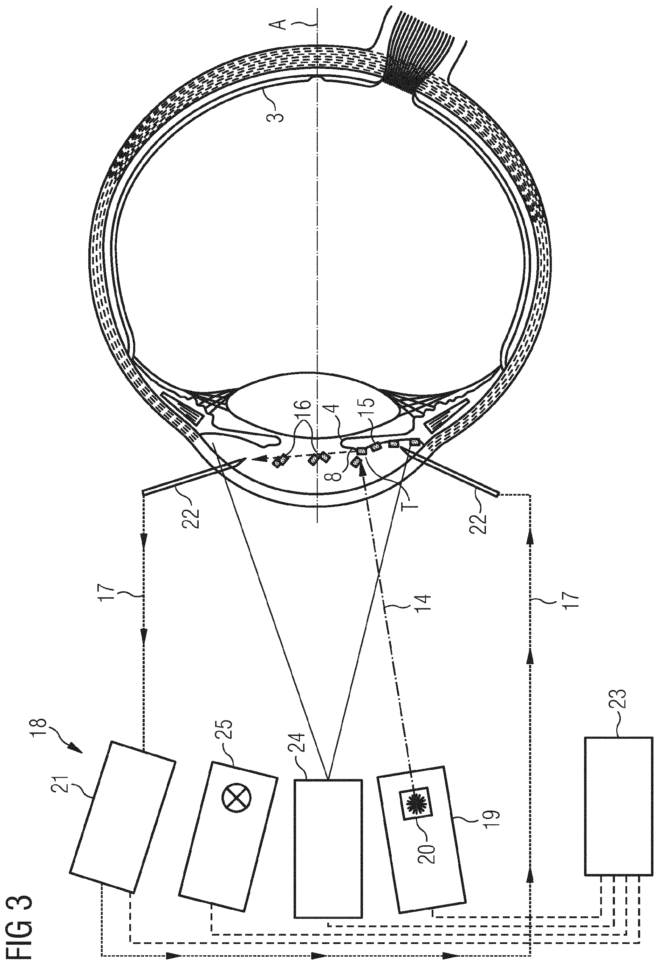

Further details of a corresponding apparatus 18 configured for and set up for carrying out a method as has been previously discussed are described below in connection with FIG. 3.

FIG. 3 shows a schematic view of the eye 1, and beyond that a schematic representation of components of the apparatus 18 which is adapted to and set up for changing the human perceptual color appearance of the iris of a human's or animal's eye.

The apparatus 18 comprises as an energy source or energy module for generating a plurality of predefined energy quantities a laser device 19 having a laser source 20 for generating predefined laser pulses 14. Via a not explicitly shown focusing device, for example involving an optical system, the laser device 19 is operable to direct and apply the generated predefined laser pulses 14 to the anterior stroma layer 8 of the iris 4. The laser device in particular may comprise a target setting device for setting a corresponding target point/area where the generated laser pulses 14 shall impinge.

The predefined laser pulses 14 are generated and applied in such a way that melanocyte cells 15 of the anterior stroma layer 8 are directly discharged (in particular: burst off), by the action of the laser pulses 14, into the aqueous humor or liquid or aqueous humor/rinsing solution mixture or rinsing solution contained in the anterior eye chamber 9.

For example, the laser device, which may for example be a Q-switched frequency doubled Nd:YAG laser, may be set up for generating laser pulses in the wavelength range of about 532 nm, having a pulse length of about 4ns, and a pulse energy of approximately 2 mJ. A pulse frequency of the laser device may lie in the range between 3 Hz and 300 Hz, for example. The focusing device and/or target setting device/unit may be adapted and set up for generating laser spot sizes in a diameter range of about 500 µm.

The apparatus further comprises a fluid pumping module 21 that is adapted and set up for maintaining the predefined mechanical flow 17 through the anterior eye chamber 9. The flow 17 may for example correspond to a continuous flow in which, via a pair of irrigator elements 22, for example, rinsing solution is supplied and drained from the anterior eye chamber 9. By the mechanical flow 17 thus generated, it is possible to remove ablated and discharged melanocytes and melanocyte material from the anterior eye chamber 9, which in particular leads to the advantages as described in more details hereinabove.

As is indicated in FIG. 3, the apparatus 18 may comprise one or more controller units 23 that is/are specifically programmed and set up for controlling the components of the apparatus 18 in accordance with any suitable method according to the invention as described herein.

As an example, the controller unit 23 may be programmed and set up such that a mechanically generated flow as described hereinabove can be maintained, such as for example in accordance with a predefined flow profile defined for at least one of the time prior to, during, and/or after applying the one or more energy quantities, e.g. laser pulses 14, to the anterior stroma layer 8 of the iris 4.

Further, the one or more controller units 23 may be coupled to the laser device 19, in order to generate and apply the predefined laser pulses 14 to the anterior stroma layer 8 of the iris 4 as described herein.

The one or more controller units 23 may be set up such that, for example by adequately controlling a focusing/target setting device, one or more of the generated laser pulses 14 are directed/applied to a predefined location/area of the anterior stroma layer 8.

As depicted in FIG. 3, the apparatus 18 may comprise a microscope and/or scanning module 24, which may be coupled to and correspondingly set up for being controlled by the one or more controller units 23, wherein the one or more controller units 23 may be programmed and set up for adequately controlling the operation of the corresponding modules 24.

The microscope and/or scanning module 24, which may in embodiments be divided into a separate microscope module and a separate scanning module, may comprise a stereoscopic microscope, that is arranged and positionable such that an operator operating the apparatus 18 is able to observe at least the anterior stroma layer 8 of the stroma 6.

The microscope may further or in the alternative be adapted such that electronic/digital imaging of eye 1, inner components thereof, in particular at least the anterior stroma layer 8, is possible/enabled.

The microscope and/or scanning module 24, for example a scanning unit, may be configured and set up for capturing an image, i.e. a digital image, of at least the iris 4 or a part thereof, for example a section that is intended for being impinged with one or more laser pulses 14 in order to change the perceptual color of that section. The captured image may be processed and used for partitioning at least a part of the surface area of the anterior stroma 8 layer into a number of surface sections.

The scanning unit may for example be programmed to define surface areas of essentially similar size and/or shape and/or pigment density. Having defined suitable/corresponding surface areas, the one or more controller units 23 may be operated to apply a respective number of predefined laser pulses 14 to one or more of the surface sections to thereby remove the melanin pigment density in accordance with a method as described herein in connection with the invention.

The one or more controller units 23 may be programmed such that the predefined surface sections are processed (in particular: impinged) with laser pulses 14, in accordance with a predefined succession of surface sections.

The predefined succession of surface sections (in particular: surface areas) may for example be determined by a partitioning module (not explicitly shown). The partitioning module may for example be programmed to determine the predetermined surface sections, e.g. their size, and/or the particular succession of surface sections within the processing sequence, on the basis of the captured image and/or on the basis of a determined density of pigments extracted for example from the captured image.

Further, the energy content/power of the energy quantities to be applied to a respective surface section may in embodiments be determined on the basis of the captured image and/or the density of pigments. Further, the specific location of the surface area on the iris 4, and/or the overall size of the iris 4, and/or the specific size of the surface sections may be used as parameters for defining and setting up characteristics of the pulses 14 to be applied to respective surface sections.

In embodiments where particular surface sections are determined, it is possible to determine or calculate at least one parameter of the mechanically generated flow on the basis of or in accordance with the specific location of a respectively processed surface section. As an example, locations near the inner border of the iris 4 defining the outer circumference of the pupil 5 may require a different flow rate/profile than surface regions lying on the outer rim of the iris 4.

Further, in case that particular surface sections are determined, parameters associated with the flow 17 and/or associated with the generation and application of the one or more energy quantities may be determined/calculated based on the particular succession of the surface sections, and/or based on the density of pigments within the surface sections, and/or based on the size of a respective surface section.

With one or more of the above options, it is in particular possible to adapt the ablation of melanin pigments to the particular configuration of the underlying iris 4, thereby reducing the risk for possible irritations of the tissue of the anterior stroma layer 8. Further, the above options, which make it possible to take account of specificities of a corresponding iris structure/shape and/or pigment density, are particularly suitable for automating the procedure of changing the perceptual color appearance of the eye.

The microscope and/or scanning module 24 may in embodiments comprise a tracking unit, or may be constructed to implement a corresponding tracking module, that is able to track the actual position, and/or shape, and/or movement of the eye 1, or one or more of the components of the eye 1 such as the iris 4, pupil 5 or others, relative to a spatial reference point.

Such a tracking function may advantageously be used for optimizing the step of applying the generated laser pulses 14, in general the generated energy quantities, to the anterior stroma layer 8. Specifically, the apparatus 18 may be configured, e.g. programmed, to track the location of the respective eye 1, and to apply the energy quantities, i.e. the laser pulses 14, in dependence of the tracking result.

For example, application of a laser pulse 14 may be inhibited (in particular: blocked) in case that the tracking result indicates that the position of the target position for the laser pulse 14 has changed, for example more than a given threshold value.

Corresponding shifts/movements in the target position may also be induced by a change in the shape of the iris 4, for example in case that the iris 4 unexpectedly dilates.

Inhibiting the application of the laser pulses 14 in case of a detected inappropriate movement of the iris 4, i.e. of the target position, may help avoiding possible damages to the anterior stroma layer 8, or other parts of the eye 1, such for example the eye's vitreous body, or even retina 3.

In embodiments, the apparatus 18, for example a focusing unit/module or the like, may be programmed such that the target location T of the energy quantity, e.g. laser pulse 14, is relocated/shifted in accordance with, in particular synchronous to, one or more of a change in position, a change in location, a change in shape, and a movement of the iris 4, or the observed component of the eye 1, respectively.

With such a setup, for example, minor shifts of the target location T, lying for example below or within a predetermined threshold, may be compensated. A corresponding threshold may be dependent on the exact location of the target location T, wherein near the inner edge of the iris 4 defining the pupil 5, the threshold may be smaller than further away from the pupil 5.

In embodiments, the apparatus 18 may further be programmed/set up, such that at least the iris 4 or sections thereof, and/or the anterior eye chamber 9 is/are scanned (in particular: recorded), for example at least during a time interval when the energy quantities, e.g. laser pulses 14, are applied to the anterior stroma layer 8.

The scanning function may be implemented with the microscope and scanning module 24, wherein it shall be mentioned that a separate scanning unit or the like may be present, and correspondingly implemented, set up, and/or programmed.

Scanning respective sections at least during application of the energy quantities may be accompanied by further operational modes based on and/or supported by the scanning result. The scanning result may for example comprise an image of the scanned section, wherein for example the respective target location T where the energy quantities impinge on the anterior stroma layer 8 may be comprised by the image and/or indicated within the image.

Implementing the scanning may be accompanied by a functionality/an operational mode in which the scanning result, e.g. a scanned or recorded image, is stored in a datastore after each predetermined number of applied energy quantities, e.g. laser pulses 14. The predetermined number may range from one to any suitable number, allowing for example the establishment of a processing history representative for the obtained color change.

Implementing the scanning may be accompanied by a functionality/an operational mode in which the actual location T of impingement, indicating a location on the anterior stroma layer 8 where the one or more energy quantities (14) actually impinged on the anterior stroma layer 8, may be determined on the basis of the scanning result.

Alternatively or in addition, the target location T may be marked (in particular: identified by a particular mark) in for example a captured image, or in the imaging result of the iris 4.

Further embodiments may involve an operational mode in which the actual locations of impingement and/or the target locations T are tracked. Corresponding results of tracking/marking may be provided for display to an operating user on a display (not shown) of the apparatus 18.

Implementing a corresponding scanning function may in embodiments involve to have a functionality in which, based on the scanning result, the flow of rinsing solution within or through the anterior eye chamber 9 (in particular: eye cavity 9) is controlled. Here, an automated adaption of the operational parameters to the respectively prevailing individual situation may be obtained.

Further, the density of pigments, in particular a local density of pigments, in particular a pigment profile, or at least a parameter representative of the density/density profile of the pigments of the anterior stroma layer 8 may be determined based on the scanning result. For example, a scanned image of the anterior stroma layer 8 may be processed for determining/identifying a description of the distribution of pigments over at least the respectively treated surface area.

Based on the determined pigment density, or the respective parameter, respectively, as has already been mentioned, the generation and/or application of one or more of the energy quantities may be controlled.

In embodiments, the apparatus 18 may be implemented, set-up, and/or programmed to be operable to generate, based on the scanning result, one or more than one display objects, and the display objects may be provided for display on a display screen of the apparatus 18 to an operator executing the method for changing the perceptual color appearance of the eye 1.

In case that a corresponding display screen (not shown) may be present, embodiments may involve that operational parameters related to the execution of the method, in particular comprising one or more of: one or more parameters related to the energy quantities, one or more target locations T and/or actual locations of incident, a first indication representative of a change, in particular local change, of the density of pigments, and a second indication representative of processed, and/or unprocessed surface areas of the anterior surface of the stroma layer 8, are displayed on the display screen, e.g. in such a way that user-guidance as regards the overall execution/performance/ implementation of the method may be obtained. User guidance in particular may improve human-machine interaction thereby further improving operational safety of the appliance 18.

In embodiments, in particular as indicated in FIG. 3, the apparatus 18 may comprise, in particular as an optional element, a slit lamp module 25, implemented as a conventional slit-lamp, and configured to be operated with the remaining components of the apparatus 18 such that an operating user and/or a corresponding microscope/scanning unit 24 is able to observe/monitor at least the anterior stroma layer 8, by means of a sheet of light generated by the slit-lamp and thrown onto the observed section of the eye 1. With this design, the laser pulses 14 may be applied substantially or mainly in a horizontal direction, meaning that the optical axis A of the eye 1 under consideration may be oriented horizontally, meaning that a corresponding person may sit upright during the change of the eye color.

The sheet of light may for example be generated by corresponding optical elements allowing the generation and projection the sheet of light.

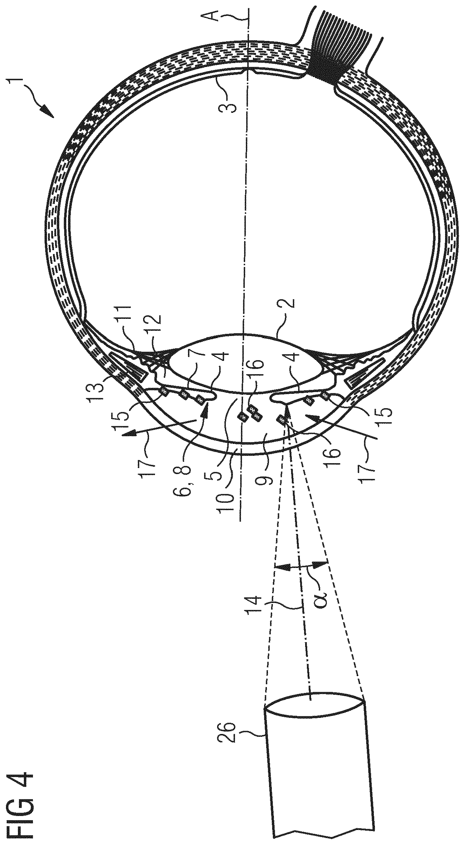

FIG. 4 shows a further representation of the human eye 1 together with further details for applying laser pulses 14 in connection with the underlying method and embodiments of the invention. Specifically, applying the laser pulses 14 may involve an optical system 26 that is implemented and adapted to generate a pulsed laser beam having a focusing angle a.

The focusing angle a may lie in the range between 10 degrees and 18 degrees, optionally between 13 degrees and 16 degrees, and may further optionally lie substantially at about 14 degrees.

The optical system may be part of a focusing device, wherein the optical system may include one or more than one lenses or lens systems for generating the focused laser beam.

Using such a focused laser beam has the advantage that the energy density at the cornea 10 can be kept comparatively small so as to avoid impairment of the cornea 10 by laser pulses passing through on their way to the anterior stroma layer 8 for ablating melanocyte cells 15.

By using the proposed focusing angles, it has been found out that the distance between the cornea 10 and the anterior stroma layer 8, being about 1.2 mm, is sufficient to have an energy density at the level of the cornea 10 that does not negatively affect the cornea 10, and to have an energy density at the anterior stroma layer 8 that is sufficient to ablate the melanocyte cells 15 or melanocyte pigments.

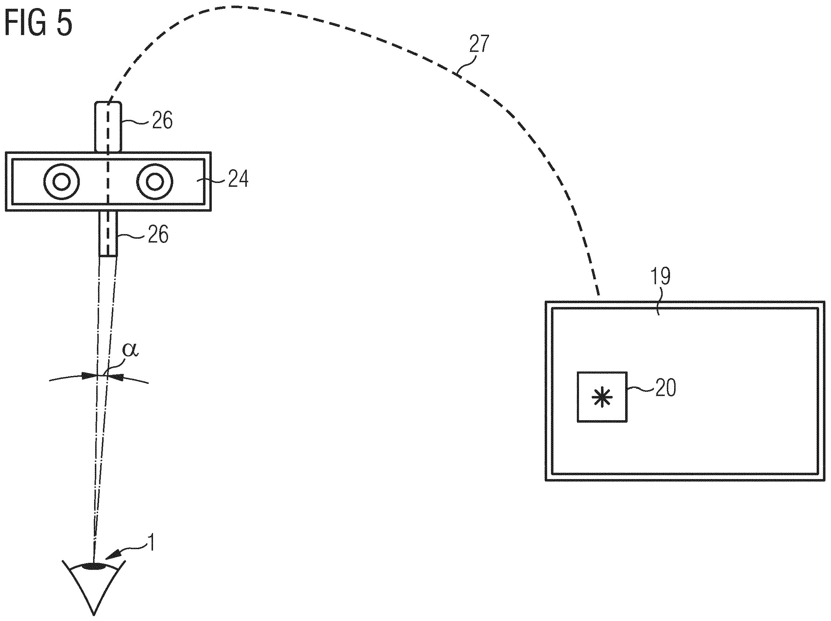

FIG. 5 shows a schematic view of the eye 1 and further details of an apparatus for carrying out the underlying invention. In the embodiment shown in FIG. 5, the laser device 19 is and may positioned and located remote from the eye 1, wherein laser pulses 14 generated by the laser device 19 are guided by a flexible fiber optical system 27 from the laser source 20 towards the optical system 26 used for focusing the laser pulses 14 under the focusing angle a, which may lie between 10 degrees and 18 degrees, onto the anterior stroma layer 8 of the eye 1.

The fiber optical system may comprise an optical fiber (not explicitly shown) with a fiber-optic core diameter lying in the range between 270 µm and 290 µm, in particular at about 280 µm.

Providing such a fiber optical system and a remote laser device 19 has the advantage that the orientation of the optical axis of the eye 1 during the procedure of changing the eye color may be selected within a comparatively broad range. In particular, the optical axis A of the eye 1 may be oriented vertically during the procedure of changing the eye color, meaning that a corresponding human being may be placed horizontally on a table, which may be advantageous for restricting overall movements of the human being, including eye and/or head movements during the procedure.

The optical system 26 may be provided in connection with a microscope 28 enabling the user and/or device applying the method to investigate the eye and/or anterior stroma layer during the whole procedure.

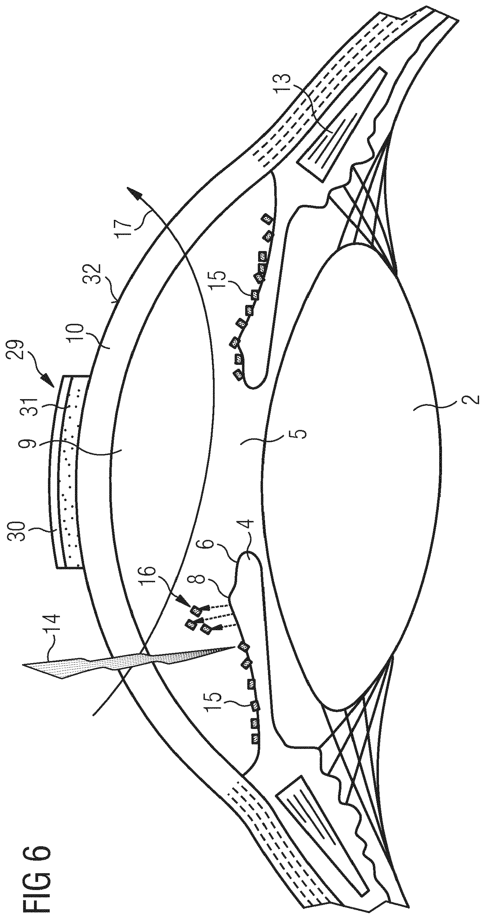

FIG. 6 shows the enlarged view of FIG. 3 together with an eye shielding element 29 which may in embodiments of the invention provided together with the proposed apparatus and together with carrying out the method.

In particular, the eye shielding element 29 may be implemented and configured to be able, when properly played on the outer layer or side of the cornea 10, to absorb energy quantities, in particular energy quants, such as laser pulses, that have been generated and directed to the eye 1 in order to ablate melanocyte cells 15 or melanocyte pigments. Such an eye shielding element 29 may be of advantage in case that a sudden eye movement shifts the actual target location of respective energy quantities off the stroma 6, meaning that such energy quantities would, without the eye shielding element 29, hit and possibly affect the retina 3, for example.

The eye shielding element 29 as shown in FIG. 6 comprises a composite structure comprising an absorption layer 30 for absorbing energy quantities 14 intended for ablating melanocyte cells 15 and impinging thereon. The eye shielding element 29 further comprises an adhesion layer 31 configured and set up for being coupled with the outer cornea layer 32 by adhesive forces. The absorption layer 30 may comprise one or more than one metallic layers, such as stainless steel layers. The adhesion layer 31 may comprise an open-pored, adhesive material layer, such as a foamed layer, for example comprising and/or made from a polyvinyl alcohol material. The foam layer may be advantageous for obtaining a stable positioning of the eye shielding element 29 on the outer cornea layer 32 which in general includes a liquid film.

The eye shielding element 29 may having a convex; and/or circular shape, and/or may have a diameter lying in the range between 0.5 mm and 3 mm, or in the range between 1 mm and 2 mm, or substantially at about 1.5 mm.

The apparatus 18 and method has been described in connection with the figures as comprising a laser device 19 for generating and applying the energy quantities. In embodiments, the apparatus 18 may comprise either as an alternative or in addition to the laser device a pressure wave generator, in particular a corresponding pressure wave generator module (not explicitly shown).

The pressure wave generator may be operable, set up and programmed to generate and emit energy quantities in the form of mechanical pressure waves, in particular shock or blast waves, towards and onto the anterior stroma layer so as to change the density of pigments in a way similar to that as described in connection with laser pulses 14. Such a pressure wave generator may be operable, set up and programmed to generate the pressure waves by induced cavitation within a liquid that is in liquid contact with the eye liquid as contained in the anterior eye chamber 9.

The induced cavitation may for example be generated by impinging a solid state target with electromagnetic waves, in particular laser light, preferably pulsed laser light, or other forms of energy such as but not restricted to ultrasound energy. In accordance with the invention, the mechanical pressure waves may be generated and applied to the anterior stroma layer 8 in such a way that melanocyte cells of the anterior stroma layer 8 are ablated in the corresponding area of impact and, as a direct response, are, at least in part, discharged into the liquid contained in the anterior eye chamber 9, such that the melanocytes, corresponding cellular material and pigment contained therein can be removed by the flow 17 maintained by the fluid pumping module 21.

The procedure of removal of the melanocyte material essentially corresponds to the removal as described in connection with laser-based ablation. The only difference is the way of applying energy of the anterior stroma layer 8. Essentially the same advantages and effects as regards the change in the perceptual color appearance of the eye 1 may be obtained. Hence, reference is made to the discussion above.

It shall be noted, that electromagnetic-based, in particular laser-based, and/or pressure-wave-based ablation may be used as alternatives or jointly, wherein the combined use may allow a finer adjustment to respective individual needs as regards the density of pigments in a respective area of interest of an iris 4 of interest.

Reference is now made to FIG. 4 showing an example flow chart for carrying out a method according to the invention. The method for changing the perceptual color appearance of the iris 4 may comprise the following steps:
- positioning 401 an eye 1 relative to a generator module 19 configured, set up and programmed for generating a plurality of predefined energy quantities 14, in particular laser pulses 14 and/or pressure waves;
- operating 402 the generator module 19 to generate the plurality of pre-defined energy quantities 14, the energy quantities 14 defined such that interaction with melanocyte cells 15 of the anterior stroma layer 8 leads to melanocyte cell ablation 16;
- directing and applying 403 the generated energy quantities 14 to the anterior stroma layer 8 of the eye 1, wherein the anterior stroma layer 8 comprises a non-zero density of melanin-pigments 15, and, by applying the energy quantities 14, discharging the melanocyte cell material at least in part into liquid contained within the anterior eye chamber 9; and
- removing 404 the melanocyte cell material 16 by maintaining a flow of rinsing liquid 17 through/within the anterior eye chamber 9.

The proposed sequence of steps in particular is efficient in removing melanin pigment from the anterior stroma layer 8 thereby shifting perceptual color appearance of the iris 4/eye 1 towards blue. The proposed method steps may be carried out at least in part in an automated manner, allowing their execution by trained medical staff.

It has been shown, that the proposed method and apparatus are well suitable in obtaining the underlying object of the invention. In particular, the proposed method as such represents a non-chirurgical method of changing eye color, wherein the proposed method, in particular each single step of the method, and the apparatus, in particular each single unit or module of the proposed apparatus, are particularly suitable for use in non-surgical treatments of the iris for changing the perceptual color appearance of the iris.

Finally it shall be noted, that the proposed method may be provided as computer-executable instructions stored on a computer-readable storage medium, wherein the computer-executable instructions are defined such that a corresponding apparatus when executing the instructions carries out the proposed method for changing eye color.

In summary, the underlying invention is directed to a method for changing the human perceptual color appearance of the iris of a human's or animal's eye by selectively decreasing the density of pigments of the anterior stroma layer of the iris, the method comprising the steps of generating a plurality of predefined energy quantities; applying one or more of the predefined energy quantities to the anterior stroma layer, wherein each of the predefined energy quantities is generated and applied such that it ablates at least in part melanocytes of the stroma, wherein the predefined energy quantities at least in part are generated and applied in such a way that ablated tissue generated as an immediate cause of the energy quantities is, at least in part, discharged directly into the anterior eye chamber such that the discharged tissue can be removed by maintaining a mechanically generated flow of rinsing solution through or within the anterior eye chamber.

### Reference Signs

- 1: eye
- 2: lens
- 3: retina
- 4: iris
- 5: pupil
- 6: stroma
- 7: pigmented epithelial cells
- 8: anterior stroma layer
- 9: anterior eye chamber
- 10: cornea
- 11: ciliar body
- 12: posterior eye chamber
- 13: Schlemm's Canal
- 14: laser pulse
- 15: melanocyte cell
- 16: discharged melanocyte cells/material
- 17: flow of rinsing solution
- 18: apparatus
- 19: laser device
- 20: laser source
- 21: fluid pumping module
- 22: irrigator elements
- 23: controller unit
- 24: microscope and scanning module
- 25: slit lamp module
- 26: optical system
- 27: fiber optical system
- 28: microscope
- 29: eye shielding element
- 30: absorption layer
- 31: adhesion layer
- 32: outer cornea layer

- 401-404: operational steps
- a: focusing angle
- A: optical axis
- T: target location

## Claims

1. Apparatus (18) specifically adapted to and set up for changing the human perceptual color appearance of the iris (4) of a human's or animal's eye (1) by selectively decreasing the density of pigments of the anterior stroma layer (8) of the iris (4), the apparatus (18) comprising:
an energy source (19, 20) comprising a generator module (19) that is adapted and set up for generating a plurality of predefined energy quantities (14) of at least one of electromagnetic type and mechanical wave type;
a focusing device (19, 20) adapted to and set up for focusing the energy quantities (14) towards the anterior stroma layer (8) of the iris (4) of the eye (1);
a fluid pumping module (21) adapted to and set up for the generation and maintenance of a predefined mechanical flow (17) of rinsing solution through, and/or within the anterior eye chamber (9); and
a controller unit (23) that is programmed and set up for:
- operating the energy source (19, 20) and focusing device (19, 20) to apply the generated, predefined energy quantities (14) to a predefined location of the anterior layer (8) of the stroma (6) in such a way that melanocyte tissue (15) of the stroma (6), that is impinged with the energy quantities (14) is ablated in such a way that it is, at least in part, discharged into the anterior eye chamber (9) as a direct cause of the interaction between the energy quantities (14) and the tissue (15); and for
- operating the fluid pumping module (21) to maintain the predefined flow (17) over a lapse of time during, and/or directly after applying the energy quantities (14) to the stroma (6), such that discharged melanocyte material (16) at least in part is fluidly discharged from the anterior chamber (9).

2. The apparatus (18) according to claim 1, further comprising one or more than one of the following:
a microscope module (24), optionally implemented as a stereoscopic microscope, arranged and positionable in such a way that an operator operating the apparatus is able to observe at least the anterior surface of the stroma layer (8) of the eye (1), the apparatus (18);
a slit lamp module (25) comprising a slit lamp and corresponding optical elements allowing the operator/supervisor of the method to throw a sheet of light into the eye (1)/onto the anterior stroma layer (8), wherein the slit lamp module (25) and microscope module (24) are optionally adapted for mutual interaction thereby implementing a slit lamp microscope for investigating at least the anterior stroma layer (8) of the iris (4);
an eye shielding element (29) implemented and set up for being coupled to the eye (1), in particular the outer cornea layer (32) in such a way to prevent energy quantities (14) from entering the posterior eye chamber (12) and/or from impinging the retina (3) of the eye (1), wherein the eye shielding element (29) having one or more than one of:
- a composite structure having an absorption layer (30) for absorbing energy quantities (14) impinging thereon, and an adhesion layer (31) for coupling with the outer cornea layer (32), in particular by adhesive and/or cohesive forces in particular to avoid the eye shielding element (29) from being floated away by lacrimal fluid, the absorption layer optionally comprising one or more than one metallic layers (30), in particular stainless steel layers, and the adhesion layer optionally comprising an open-pored material layer (31), in particular foamed layer, preferably comprising and/or made from a polyvinyl alcohol material;
- a convex; and/or circular shape, and/or a diameter lying in the range between 0.5 mm and 3 mm, or in the range between 1 mm and 2 mm, or substantially at about 1.5 mm.

3. The apparatus (18) according to at least one of claim 1 and 2, the energy source (19, 20) comprising as generator module a light emitting element (20), in particular a laser device preferably a Q-switched laser device, more preferably a Q-switched frequency doubled laser device , yet more preferably a Nd:YAG laser device, the laser device (20) operable to generate at least some of the predefined energy quantities (14), and emit the generated energy quantities (14) in the form of light pulses (14) towards and onto the anterior stroma layer (8) so as to change the density of pigments, wherein the light emitting element (19), in particular the laser device (19), optionally being operable or implemented to at least one of:
emit light, in particular pulsed laser light, in a wavelength range between 488 nm and 580 nm, in particular 532 nm; or in a wavelength range between 976 nm to 1160 nm, or in a wavelength range between 1044 to 1082 nm, or with a wavelength corresponding substantially to 1064 nm;
generate light pulses (14) having a duration in the range between 2 ns and 6 ns, in particular 4 ns;
generate light pulses (14) having an energy of about 2 mJ;
generate light pulses (14) with a pulse frequency in the range between 3 Hz and 300 Hz;
generate light pulses (14) having a peak power ranging between 0.1 MW to 0.5 MW;
generate a laser spot having a diameter ranging between 200 µm and 600 µm, preferably in the range of about 400 µm;
the energy source (19, 20) optionally comprising:
- an optical system (26) implemented and set up for generating a pulsed laser beam having a focusing angle (a) lying in the range between 10 degrees and 18 degrees, optionally between 13 degrees and 16 degrees, further optionally lying substantially at about 14 degrees, the optical system (26) optionally implemented and set up for applying the energy quantities (14) to the anterior stroma layer (8) substantially in vertical direction;
- a fiber optical system (27) implemented and set up for guiding energy quantities (14) generated by light emitting element (20) towards the target location (T), the fiber optical system (27) optionally comprising an optical fiber having a fiber-optic core diameter lying in the range between 270 µm and 290 µm, in particular at about 280 µm, wherein the fiber optical system (27) is optionally coupled between the light emitting element (20) and the optical system (26) for guiding energy quantities (14) from the light emitting element (20) located at a remote location to the optical system (26).

4. The apparatus (18) according to claim 1 or 2, the energy source comprising as generator module
a pressure wave generator, the pressure wave generator operable to generate at least some of the predefined energy quantities, and emit the generated energy quantities in the form of mechanical pressure waves, in particular shock or blast waves, towards and onto the anterior stroma layer (8) so as to change the density of pigments, wherein the pressure wave generator optionally being operable or implemented to:
generate the pressure waves by induced cavitation within a liquid that is in liquid contact with the eye liquid as contained in the anterior eye chamber (9);
the induced cavitation optionally being generated by impinging a target object with electromagnetic waves, in particular laser light, preferably pulsed laser light, and/or by an ultrasound source, in particular a piezo generator.

5. Computer-readable non-transitory storage medium comprising executable instructions which, when executed on a computer or controller-unit (23) cause the computer or controller-unit (23) to cause the apparatus (18) according to any of claims 1 to 4 to carry out a method for changing the human perceptual color appearance of the iris (4) of a human's or animal's eye (1) by selectively decreasing the density of pigments (15) of the anterior stroma layer (8) of the iris (4), the method comprising the steps of:
- generating (402), by a generator module (19, 20), a plurality of predefined energy quantities (14); and
- applying (403), by the generator module (19, 20), one or more of the predefined energy quantities (14) to the anterior stroma layer (8);
- each of the predefined energy quantities (14) being generated and applied, such that they ablate, at least in part, melanocytes (15) of the stroma (6) whilst leaving non-melanocyte tissue of at least the stroma (6) essentially undamaged;
- the predefined energy quantities (14) at least in part generated and applied in such a way that ablated tissue (16) or pigment debris, that is generated as an immediate cause of one or more of the applied energy quantities (14), is discharged into the anterior eye chamber (9), such that the discharged tissue (16) is removeable by a mechanically generated flow (17) of rinsing solution through or within the anterior eye chamber (9).

6. The computer-readable non-transitory storage medium of claim 5 comprising further executable instructions which, when executed by the controller unit (23) causes the apparatus (18) to:
provide a mechanically generated flow (17) of rinsing solution through or within the anterior eye chamber (9), and thereby
remove the tissue/pigment debris (16) from eye (1) by means of the generated flow (17).

7. The computer-readable non-transitory storage medium of claim 5 or 6 comprising further executable instructions which, when executed by the controller unit (23) causes the apparatus (18) to carry out one or more than one of the following steps:
- maintaining the mechanically generated flow (17) for a respectively predetermined lapse of time at least during, and/or after applying the predefined quantity of energy (14) to the anterior stroma layer (8);
- maintaining the mechanically generated flow (17) also during a predefined lapse of time prior to applying the predefined quantity of energy (14) to the anterior stroma layer (8);
- maintaining the mechanically generated flow (17) for at least one predetermined lapse of time in accordance with a respective, predetermined flow rate profile, the predetermined flow rate profile preferably being constant over time, at least for one, optionally for each, lapse of time, wherein at least one of a start and end point of at least one lapse of time optionally being triggered by the generating, and/or applying the predefined quantity of energy (14); wherein
- the mechanically generated flow (17) optionally comprises, at least during a predetermined first period of time, a laminar flow, and/or at least during a predetermined second period of time a turbulent flow.

8. The computer-readable non-transitory storage medium of at least one of claims 5 to 7 comprising further executable instructions which, when executed by the controller unit (23) causes the apparatus (18) to carry out the following steps:
- partitioning, preferably by a partitioning module (24), further preferably based on a captured image of the iris (4), at least a part of the surface area of the anterior stroma layer (8) into a number of predefined surface sections, preferably having a predetermined size, and
- applying a respective number of predefined energy quantities (14) to one or more surface sections;
the predefined surface sections optionally processed in accordance with a predefined succession of surface sections, the predefined succession preferably determined by the partitioning module (24);
the predetermined surface sections, in particular the size of one or more of the predetermined surface sections, and/or the particular succession of surface sections within the processing sequence, and/or the energy content/power of the energy quantity optionally being determined on the basis of the density of pigments, and/or the specific location of the surface area on the iris (4), and/or the overall size of the iris (4); wherein at least one parameter of the mechanically generated flow (17) is optionally determined on the basis of one or more than one of:
- the specific location of a respectively processed surface section,
- the particular succession of the surface sections,
- the density of pigments,
- the size of a respective surface section,
- one or more than one parameter related to generating and/or applying the energy quantities (14).

9. The computer-readable non-transitory storage medium of at least one of claims 5 to 8 comprising further executable instructions which, when executed by the controller unit (23) causes the apparatus (18) to carry out the following steps, wherein: at least one or more than one of the predefined energy quantities (14) is generated and applied to the stroma layer (8) as at least one of:
- one or more electromagnetic waves (14), in particular electromagnetic wave pulses (14), the electromagnetic waves preferably generated by a laser device (19, 20), in particular a pulsed laser device (19, 20), more particularly by a Q-switched laser device (19, 20), in particular Q-switched frequency doubled laser device, and
- one or more mechanical pressure waves, in particular shock or blast waves, preferably induced by cavitation, in particular plasma-induced cavitation, more particularly directly induced by a plasma blast, and/or induced by an electromagnetic wave pulse, the mechanical pressure waves generated within liquid contained in or in direct fluid contact with the anterior eye chamber (9),
and/or comprising further instructions which, when executed by the controller unit (23) cause the apparatus (18) to:
determine the local pigment density, and/or thickness of melanocyte layer at the anterior stroma layer (8), wherein generating, and/or locally applying one or more of the energy quantities (14) is carried out in dependence of a respective local density of pigments, and/or local thickness of the melanocyte layer of the anterior stroma layer (8).

10. The computer-readable non-transitory storage medium of at least one of claims 5 to 9 comprising further executable instructions which, when executed by the controller unit (23) causes the apparatus (18) to generate at least one or more than one, in particular substantially all, of the energy quantities (14) as electromagnetic wave pulses (14), in particular for direct application to the iris (4) tissue, by operating a laser device (19, 20) to:
- generate at least a part of the energy quantities (14) to have a wavelength range between 488 nm to 580 nm, or in a wavelength range between 522 to 541 nm, or with a wavelength corresponding substantially to 532 nm, or in a wavelength range between 976 nm to 1160 nm, or in a wavelength range between 1044 to 1082 nm, or with a wavelength corresponding substantially to 1064 nm; and/or
- generate at least a part of the energy quantities (14) with a pulse frequency in the range between 3 Hz and 300 Hz;
and/or
- generate at least a part of the energy quantities (14) to have a pulse length lying in the range between 2 ns to 6 ns, or corresponding substantially to 4 ns;
and/or
- generate, by operating or adjusting an optical system (26) of the laser device (19, 20), a pulsed laser beam for generating at least one or more than one, in particular substantially all, of the energy quantities (14), the pulsed laser beam having a focusing angle (a) lying in the range between 10 degrees and 18 degrees, optionally between 13 degrees and 16 degrees, further optionally lying substantially at about 14 degrees; wherein the energy quantities (14) are guided from a light emitting element (20) to the optical system (26) via a fiber optical system (27), preferably having an optical fiber with a fiber-optic core diameter lying in the range between 270 µm and 290 µm, in particular at about 280 µm;
and/or
- apply the energy quantities (14) to the anterior stroma layer (8) substantially in vertical direction;
and/or
to generate at least one or more than one, in particular substantially all, of the energy quantities (14) as mechanical wave pulses as a direct cause of a plasma-induced burst pulse generated at least in part by one or more than one of:
- a direct interaction of a laser pulse with a laser target that is in fluid communication with the intraocular humor;
- a direct interaction of a laser pulse with a laser target that is placed within the anterior eye chamber (9);
- a direct interaction of a laser pulse with rinsing-solution within the anterior eye chamber (9); and
- a direct interaction of a laser pulse with the anterior stroma layer (8), in particular fibrovascular stroma structure of the stroma layer (8).

11. The computer-readable non-transitory storage medium of at least one of claims 5 to 10 comprising further executable instructions which, when executed by the controller unit (23) causes the apparatus (18) to:
track, by a tracking module (24), in particular an optical tracking module (24), one or more than one of a position, shape, and movement of the eye (1) or one of the components of the eye (1), such as the iris (4) or the pupil (5), relative to a spatial reference point, and
apply, at least in part, the generated predefined energy quantities (14), optionally each of the generated predefined energy quantities (14), in dependence on the tracking result;
and optionally further comprising executable instructions which, when executed by the controller unit (23) cause the apparatus to:
- inhibit the generator module and/or inhibiting application of one or more than one energy quantity (14) in case that the tracking result indicates one or more of a change in position, a change in location, a change in shape, and movement, and/or
- relocate the target setting of the energy quantity in accordance with one or more of a change in position, a change in location, a change in shape, and a movement; and/or
- couple, prior to applying one or more than one of the energy quantities (14), an eye shielding element (29) to the eye (1), in particular to the outer layer (32) of the cornea (10);
the eye shielding element (29) implemented and positioned to prevent energy quantities (14) from entering the posterior eye chamber (12) and/or from impinging the retina (3) of the eye (1);
the eye shielding element (29) optionally being implemented in a composite structure comprising an absorption layer (30) for absorbing energy quantities (14) impinging thereon, and an adhesion layer (31) for coupling with the outer cornea layer (32), in particular by adhesive forces, the absorption layer (30) optionally comprising one or more than one metallic layers (30), in particular stainless steel layers, and the adhesion layer (31) optionally comprising an open-pored material layer (31), in particular foamed layer, preferably comprising and/or made from a polyvinyl alcohol material;
the eye shielding element (29) being partly, in particular in the adhesion layer (31), filled with or absorbing natural liquid from the outer side of the cornea thereby adhering to the cornea like for instance a paper tissue or blotting paper would,
the eye shielding element (29) optionally having a convex; and/or circular shape, and/or a diameter lying in the range between 0.5 mm and 3 mm, or in the range between 1 mm and 2 mm, or substantially at about 1.5 mm.

12. The computer-readable non-transitory storage medium of at least one of claims 5 to 11 comprising further executable instructions which, when executed by the controller unit (23) causes the apparatus (18) to:
scan, by a scanning module (24), at least the iris (4) or sections thereof, and/or the anterior eye chamber (9) at least during application of the energy quantities (14); and performing one or more than one of the following steps:
- determining a shape of the iris and/or a track, pathway and/or succession of target points to be impinged with the energy quantities based on the scanning result;
- storing the scanning result after each predetermined number of applied energy quantities;
- determining, based on the scanning result, an actual location of impingement or an actual averaged location of impingement respectively indicating an actual location on the anterior stroma layer (8)/the iris (4) where one or more energy quantities (4) indeed impinged on the anterior stroma layer (8), and optionally track the target locations of impingement;
- controlling, based on the scanning result, the flow (17) of rinsing solution within or through the anterior eye cavity (9);
- based on the scanning result, determining a density of pigments, in particular a local density of pigments, in particular a pigment profile, or at least a parameter representative of the density, in particular the local density, of pigments may be determined based on the scanning result, and controlling the generation and/or application of one or more of the energy quantities (14) based at least in part on the density of pigments or the respective parameter;
- based on the scanning result, determining a change, in particular local change, in the density of pigments, or at least a parameter representative of the change in density of pigments in the anterior stroma layer (8), and controlling the generation and/or application of the energy quantities (14) based on the determined change of the density of pigments or the respective parameter;
- generating, based on the scanning result, one or more than one display objects for display on a display screen to an operator executing the method; and optionally providing for display on the display screen operational parameters related to the execution of the method, in particular comprising one or more than one of: one or more than one parameter related to the energy quantities (14), one or more points of impact (T) of one or more applied energy quantities (14) on the anterior stroma layer (8), in particular one or more of a one or more past and future points of impact, of energy quantities (14), a first indication representative of a change, in particular local change, of the density of pigments, and a second indication representative of processed, and/or unprocessed surface areas of the anterior surface of the stroma layer.

13. The Apparatus (18) according to any of claims 1 to 4, comprising the computer-readable non-transitory storage medium according to any of claims 5 to 12.

14. Controller-unit (23) comprising executable instructions which, when executed on the controller-unit (23) cause the controller-unit (23) to cause the apparatus (18) according to any of claims 1 to 4 to carry out a method for changing the human perceptual color appearance of the iris (4) of a human's or animal's eye (1) by selectively decreasing the density of pigments (15) of the anterior stroma layer (8) of the iris (4), the method comprising the steps of:
- generating (402), by a generator module (19, 20), a plurality of predefined energy quantities (14); and
- applying (403), by the generator module (19, 20), one or more of the predefined energy quantities (14) to the anterior stroma layer (8);
- each of the predefined energy quantities (14) being generated and applied, such that they ablate, at least in part, melanocytes (15) of the stroma (6) whilst leaving non-melanocyte tissue of at least the stroma (6) essentially undamaged;
- the predefined energy quantities (14) at least in part generated and applied in such a way that ablated tissue (16) or pigment debris, that is generated as an immediate cause of one or more of the applied energy quantities (14), is discharged into the anterior eye chamber (9), such that the discharged tissue (16) is removeable by a mechanically generated flow (17) of rinsing solution through or within the anterior eye chamber (9).

## Patentansprüche

1. Gerät (18), das spezifisch dazu ausgelegt und eingerichtet ist, die humane wahrnehmende Farberscheinung der Iris (4) eines Auges (1) eines Menschen oder eines Tiers durch selektives Verringern der Dichte von Pigmenten der anterioren Stromaschicht (8) der Iris (4) zu ändern, wobei das Gerät (18) umfasst:
eine Energiequelle (19, 20), die ein Generatormodul (19) umfasst, das dazu ausgelegt und eingerichtet ist, eine Mehrzahl vorbestimmter Energiemengen (14) zumindest eines elektromagnetischen Typs und eines Mechanische-Wellen-Typs zu erzeugen;
eine Fokussiervorrichtung (19, 20), die dazu ausgelegt und eingerichtet ist, die Energiemengen (14) hin zu der anterioren Stromschicht (8) der Iris (4) des Auges (1) zu fokussieren;
ein Fluidpumpmodul (21), das dazu ausgelegt und eingerichtet ist, eine vorbestimmte mechanische Strömung (17) einer Spüllösung durch die anteriore Augenkammer (9) und/oder innerhalb dieser zu erzeugen und aufrechtzuerhalten; und
eine Steuereinheit (23), die programmiert und eingerichtet ist zum:
Betreiben der Energiequelle (19, 20) und der Fokussiervorrichtung (19, 20), um die erzeugten, vorbestimmten Energiemengen (14) an eine vorbestimmte Stelle der anterioren Schicht (8) der Stroma (6) aufzuwenden, so dass Melanozytengewebe (15) der Stroma (6), auf das die Energiemengen (14) auftreffen, so ablatiert wird, dass es als direkte Ursache der Interaktion zwischen den Energiemengen (14) und dem Gewebe (15) zumindest teilweise in die anteriore Augenkammer (9) ausgeschieden wird;
und zum
Betreiben des Fluidpumpmoduls (21), um die vorbestimmte Strömung (17) über eine Zeitdauer während des Anwendens der Energiemengen (14) an das Stroma (6) und/oder direkt danach aufrechtzuerhalten, so dass ausgeschiedenes Melanozytenmaterial (16) zumindest teilweise aus der anterioren Kammer (9) fluidausgeschieden wird.

2. Gerät (18) nach Anspruch 1, das ferner eines oder mehr als eines des Folgenden umfasst:
ein Mikroskopmodul (24), das optional als stereoskopisches Mikroskop umgesetzt ist, das so angeordnet und positionierbar ist, dass ein Benutzer, der das Gerät bedient, in der Lage ist, zumindest die anteriore Oberfläche der Stromaschicht (8) des Auges (1) zu beobachten, das Gerät (18);
ein Spaltlampenmodul (25), das eine Spaltlampe und entsprechende optische Elemente umfasst, die es dem Benutzer des Verfahrens/der Aufsichtsperson über dieses ermöglichen, eine Lichtscheibe in das Auge (1)/auf die anteriore Stromaschicht (8) zu werfen, wobei das Spaltlampenmodul (25) und das Mikroskopmodul (24) optional für eine gegenseitige Interaktion ausgelegt sind, wodurch ein Spaltlampenmikroskop zum Untersuchen zumindest der anterioren Stromaschicht (8) der Iris (4) umgesetzt wird;
ein Augenabschirmungselement (29), das dazu umgesetzt und eingerichtet ist, mit dem Auge (1), insbesondere der äußeren Hornhautschicht (32), verbunden zu werden, um zu verhindern, dass Energiemengen (14) in die posteriore Augenkammer (12) eintreten und/oder auf die Netzhaut (3) des Auges (1) auftreffen,
wobei das Augenabschirmungselement (29) eines oder mehr als eines aufweist von:
einer Verbundstruktur mit einer Absorptionsschicht (30) zum Absorbieren von Energiemengen (14), die auf diese auftreffen, und eine Adhäsionsschicht (31) zur Verbindung mit der äußeren Hornhautschicht (32), insbesondere durch Adhäsions- und/oder Kohäsionskräfte, insbesondere um zu vermeiden, dass das Augenabschirmungselement (29) durch Tränenflüssigkeit weggetrieben wird, wobei die Absorptionsschicht optional eine oder mehr als eine Metallschicht (30), insbesondere Edelstahlschichten, umfasst, und wobei die Adhäsionsschicht optional eine Schicht (31) offenporigen Materials, insbesondere eine geschäumte Schicht, umfasst, die vorzugsweise ein Polyvinylalkoholmaterial umfasst und/oder daraus hergestellt ist;
einer konvexen; und/oder kreisförmigen Form und/oder einem Durchmesser, der im Bereich zwischen 0,5 mm und 3 mm oder im Bereich zwischen 1 mm und 2 mm oder im Wesentlichen bei ungefähr 1,5 mm liegt.

3. Gerät (18) nach zumindest einem von Anspruch 1 und 2, wobei die Energiequelle (19, 20) als Generatormodul ein lichtemittierendes Element (20), insbesondere eine Laservorrichtung, vorzugsweise eine gütegeschaltete Laservorrichtung, mehr bevorzugt eine gütegeschaltete frequenzverdoppelte Laservorrichtung, noch bevorzugter eine Nd:YAG-Laservorrichtung, umfasst, wobei die Laservorrichtung (20) betreibbar ist, um zumindest gewisse der vorbestimmten Energiemengen (14) zu erzeugen und die erzeugten Energiemengen (14) in Form von Lichtimpulsen (14) hin zur anterioren Stromaschicht (8) und auf diese zu emittieren, so dass die Dichte von Pigmenten verändert wird, wobei das lichtemittierende Element (19), insbesondere die Laservorrichtung (19), optional betreibbar oder umgesetzt ist zu zumindest einem von:
Emittieren von Licht, insbesondere gepulstem Laserschicht, in einem Wellenlängenbereich zwischen 488 nm und 580 nm, insbesondere 532 nm; oder
in einem Wellenlängenbereich zwischen 976 nm und 1160 nm oder in einem Wellenlängenbereich zwischen 1044 und 1082 nm oder mit einer Wellenlänge, die im Wesentlichen 1064 nm entspricht;
Erzeugen von Lichtimpulsen (14) mit einer Dauer im Bereich zwischen 2 ns und 6 ns, insbesondere 4 ns;
Erzeugen von Lichtimpulsen (14) mit einer Energie von ungefähr 2 mJ;
Erzeugen von Lichtimpulsen (14) mit einer Impulsfrequenz im Bereich zwischen 3 Hz und 300 Hz;
Erzeugen von Lichtimpulsen (14) mit einer Peakleistung im Bereich zwischen 0,1 MW und 0,5 MW;
Erzeugen eines Laserpunkts mit einem Durchmesser im Bereich zwischen 200 pm und 600 µm, vorzugsweise im Bereich von ungefähr 400 µm; wobei die Energiequelle (19, 20) optional umfasst:
ein optisches System (26), das dazu umgesetzt und eingerichtet ist, einen gepulsten Laserstrahl mit einem Fokussierwinkel (a) zu erzeugen, der im Bereich zwischen 10 Grad und 18 Grad, optional zwischen 13 Grad und 16 Grad, liegt, der ferner optional im Wesentlichen bei ungefähr 14 Grad liegt, wobei das optische System (26) optional dazu umgesetzt und eingerichtet ist, die Energiemengen (14) auf die anteriore Stromaschicht (8) im Wesentlichen in vertikaler Richtung anzulegen;
ein optisches Fasersystem (27), das dazu umgesetzt und eingerichtet ist, Energiemengen (14), die vom lichtemittierenden Element (20) erzeugt werden, hin zur Zielstelle (T) zu leiten, wobei das optische Fasersystem (27) optional eine optische Faser mit einem Kerndurchmesser der optischen Faser im Bereich zwischen 270 µm und 290 µm, insbesondere bei ungefähr 280 µm, umfasst, wobei das optische Fasersystem (27) optional zwischen dem lichtemittierenden Element (20) und dem optischen System (26) verbunden ist, um Energiemengen (14) vom lichtemittierenden Element (20), das sich an einer entfernten Stelle befindet, zum optischen System (26) zu leiten.

4. Gerät (18) nach Anspruch 1 oder 2, wobei die Energiequelle als Generatormodul umfasst:
einen Druckwellengenerator, wobei der Druckwellengenerator betreibbar ist, um zumindest gewisse der vorbestimmten Energiemengen zu erzeugen und die erzeugten Energiemengen in Form von mechanischen Druckwellen, insbesondere Schock- oder Detonationswellen, hin zur anterioren Stromaschicht (8) und auf diese zu emittieren, so dass die Dichte von Pigmenten verändert wird, wobei der Druckwellengenerator optional betreibbar oder umgesetzt ist zum:
Erzeugen der Druckwellen durch induzierte Kavitation innerhalb einer Flüssigkeit, die mit der Augenflüssigkeit, wie sie in der anterioren Augenkammer (9) enthalten ist, in Flüssigkontakt steht;
wobei die induzierte Kavitation optional dadurch erzeugt wird, dass elektromagnetische Wellen, insbesondere Laserlicht, vorzugsweise gepulstes Laserlicht, auf ein Zielobjekt gestoßen werden, und/oder durch eine Ultraschallquelle, insbesondere einen Piezogenerator, auf ein Zielobjekt auftreffen.

5. Computerlesbares nicht flüchtiges Speichermedium, das ausführbare Anweisungen umfasst, die, wenn sie auf einem Computer oder einer Steuereinheit (23) ausgeführt werden, den Computer oder die Steuereinheit (23) dazu veranlassen, das Gerät (18) nach einem der Ansprüche 1 bis 4 dazu zu veranlassen, ein Verfahren zum Ändern der humanen wahrnehmenden Farberscheinung der Iris (4) eines Auges (1) eines Menschen oder eines Tiers durch selektives Verringern der Dichte von Pigmenten (15) der anterioren Stromaschicht (8) der Iris (4) durchzuführen, wobei das Verfahren die Schritte umfasst:
Erzeugen (402) einer Mehrzahl vorbestimmter Energiemengen (14) durch ein Generatormodul (19, 20); und
Anwenden (403) einer oder mehrerer der vorbestimmten Energiemengen (14) durch das Generatormodul (19, 20) auf die anteriore Stromaschicht (8);
wobei jede der vorbestimmten Energiemengen (14) so erzeugt und angewandt wird, dass sie Melanozyten (15) der Stroma (6) zumindest teilweise ablatieren, während Nicht-Melanozytengewebe zumindest der Stroma (6) im Wesentlichen unbeschädigt bleibt;
wobei die vorbestimmten Energiemengen (14) zumindest teilweise so erzeugt und angewandt werden, dass ablatierte Trümmer von Gewebe (16) oder Pigment, wie als unmittelbare Ursache einer oder mehrerer der angewandten Energiemengen (14) erzeugt, in die anteriore Augenkammer (9) ausgeschieden wird, so dass das ausgeschiedene Gewebe (16) durch eine mechanisch erzeugte Strömung (17) einer Spüllösung durch die anteriore Augenkammer (9) oder innerhalb dieser entfernbar ist.

6. Computerlesbares nicht flüchtiges Speichermedium nach Anspruch 5, das ferner ausführbare Anweisungen umfasst, die, wenn sie von der Steuereinheit (23) ausgeführt werden, das Gerät (18) veranlassen zum:
Bereitstellen einer mechanisch erzeugten Strömung (17) einer Spüllösung durch die anteriore Augenkammer (9) oder innerhalb dieser, und dadurch Entfernen von Gewebe-/Pigmenttrümmer (16) aus dem Auge (1) mithilfe der erzeugten Strömung (17).

7. Computerlesbares nicht flüchtiges Speichermedium nach Anspruch 5 oder 6, das ferner ausführbare Anweisungen umfasst, die, wenn sie von der Steuereinheit (23) ausgeführt werden, das Gerät (18) dazu veranlassen, einen oder mehr als einen der folgenden Schritte durchzuführen:
Aufrechterhalten der mechanisch erzeugten Strömung (17) für eine jeweils vorbestimmte Zeitdauer zumindest während des Anwendens der vorbestimmten Menge an Energie (14) an die anteriore Stromaschicht (8) und/oder danach;
Aufrechterhalten der mechanisch erzeugten Strömung (17) auch während einer vorbestimmten Zeitdauer vor Anwenden der vorbestimmten Menge an Energie (14) an die anteriore Stromaschicht (8);
Aufrechterhalten der mechanisch erzeugten Strömung (17) für zumindest eine vorbestimmte Zeitdauer gemäß einem jeweiligen, vorbestimmten Durchflussratenprofil, wobei das vorbestimmte Durchflussratenprofil vorzugsweise über die Zeit hinweg für zumindest eine, optional für jede, Zeitdauer konstant ist, wobei zumindest eines von einem Start- und Endpunkt zumindest einer Zeitdauer optional durch das Erzeugen und/oder Anwenden der vorbestimmten Menge an Energie (14) ausgelöst wird; wobei:
die mechanisch erzeugte Strömung (17) optional zumindest während eines ersten vorbestimmten Zeitraums eine Laminarströmung und/oder eine turbulente Strömung zumindest während eines vorbestimmten zweiten Zeitraums umfasst.

8. Computerlesbares nicht flüchtiges Speichermedium nach zumindest einem der Ansprüche 5 bis 7, das ferner ausführbare Anweisungen umfasst, die, wenn sie von der Steuereinheit (23) ausgeführt werden, das Gerät (18) dazu veranlassen, die folgenden Schritte durchzuführen:
Teilen zumindest eines Teils des Oberflächenbereichs der anterioren Stromaschicht (8) in eine Anzahl vorbestimmter Oberflächenabschnitte, die vorzugsweise eine vorbestimmte Größe aufweisen, vorzugsweise durch ein Teilungsmodul (24), ferner vorzugsweise auf Basis eines aufgenommenen Bilds der Iris (4), und Anwenden einer jeweiligen Anzahl vorbestimmter Energiemengen (14) auf einen oder mehrere Oberflächenabschnitte;
wobei die vorbestimmten Oberflächenabschnitte optional gemäß einer vorbestimmten Abfolge von Oberflächenabschnitten verarbeitet werden, die vorbestimmte Abfolge vorzugsweise durch das Teilungsmodul (24) bestimmt wird;
wobei die vorbestimmten Oberflächenabschnitte, insbesondere die Größe eines oder mehrerer der vorbestimmten Oberflächenabschnitte, und/oder die spezielle Abfolge von Oberflächenabschnitten innerhalb der Verarbeitungssequenz und/oder der Energiegehalt/die Leistung der Energiemenge optional auf Basis der Dichte von Pigmenten und/oder der spezifischen Stelle des Oberflächenbereichs auf der Iris (4) und/oder der Gesamtgröße der Iris (4) bestimmt wird;
wobei zumindest ein Parameter der mechanisch erzeugten Strömung (17) optional auf Basis eines oder mehr als einem bestimmt wird von:
der spezifischen Stelle eines jeweils verarbeiteten Oberflächenabschnitts, der bestimmten Abfolge der Oberflächenabschnitte, der Dichte von Pigmenten, der Größe eines jeweiligen Oberflächenabschnitts, einem oder mehr als einem Parameter in Zusammenhang mit dem Erzeugen und/oder Anwenden der Energiemengen (14).

9. Computerlesbares nicht flüchtiges Speichermedium nach zumindest einem der Ansprüche 5 bis 8, das ferner ausführbare Anweisungen umfasst, die, wenn sie von der Steuereinheit (23) ausgeführt werden, das Gerät (18) dazu veranlassen, die folgenden Schritte durchzuführen, wobei: zumindest eine oder mehr als eine der vorbestimmten Energiemengen (14) als zumindest eines erzeugt und auf die Stromaschicht (8) angewandt wird von:
einer oder mehreren elektromagnetischen Wellen (14), insbesondere elektromagnetischen Wellenimpulsen (14), wobei die elektromagnetischen Wellen vorzugsweise von einer Laservorrichtung (19, 20), insbesondere einer gepulsten Laservorrichtung (19, 20), im Speziellen von einer gütegeschalteten Laservorrichtung (19, 20), insbesondere einer gütegeschalteten frequenzgedoppelten Laservorrichtung, erzeugt werden, und
eine oder mehrere mechanische Druckwellen, insbesondere Schock- oder Detonationswellen, die vorzugsweise durch Kavitation, insbesondere plasmainduzierte Kavitation, induziert werden, im Speziellen direkt durch eine Plasmaexplosion induziert werden und/oder durch einen elektromagnetischen Wellenimpuls induziert werden, wobei die mechanischen Druckwellen innerhalb einer Flüssigkeit erzeugt werden, die in der anterioren Augenkammer (9) enthalten oder in direktem Fluidkontakt mit dieser steht,
und/oder ferner umfassend Anweisungen, die, wenn sie von der Steuereinheit (23) ausgeführt werden, das Gerät (18) veranlassen zum:
Bestimmen der lokalen Pigmentdichte und/oder Dicke einer Melanozytenschicht an der anterioren Stromaschicht (8), wobei das Erzeugen und/oder das lokale Anwenden einer oder mehrerer der Energiemengen (14) abhängig von einer jeweiligen lokalen Dichte von Pigmenten und/oder lokalen Dicke der Melanozytenschicht der anterioren Stromaschicht (8) durchgeführt wird.

10. Computerlesbares nicht flüchtiges Speichermedium nach zumindest einem der Ansprüche 5 bis 9, das ferner ausführbare Anweisungen umfasst, die, wenn sie von der Steuereinheit (23) ausgeführt werden, das Gerät (18) dazu veranlassen, zumindest eine oder mehr als eine, insbesondere im Wesentlichen alle, der Energiemengen (14) als elektromagnetische Wellenimpulse (14) zu erzeugen, insbesondere für eine direkte Anwendung auf das Gewebe der Iris (4), indem eine Laservorrichtung (19, 20) betrieben wird zum:
Erzeugen zumindest eines Teils der Energiemengen (14), so dass diese einen Wellenlängenbereich zwischen 488 nm und 580 nm aufweisen, oder in einem Wellenlängenbereich zwischen 522 und 541 nm oder mit einer Wellenlänge, die im Wesentlichen 532 nm entspricht, oder in einem Wellenlängenbereich zwischen 976 nm und 1160 nm oder in einem Wellenlängenbereich zwischen 1044 und 1082 nm oder mit einer Wellenlänge, die im Wesentlichen 1064 nm entspricht, und/oder
Erzeugen zumindest eines Teils der Energiemengen (14) mit einer Impulsfrequenz im Bereich zwischen 3 Hz und 300 Hz;
und/oder
Erzeugen zumindest eines Teils der Energiemengen (14), so dass diese eine Impulslänge aufweist, die im Bereich zwischen 2 ns und 6 ns liegt oder im Wesentlichen 4 ns entspricht;
und/oder
Erzeugen eines gepulsten Laserstrahls durch Betreiben oder Einstellen eines optischen System (26) der Laservorrichtung (19, 20), um zumindest eine oder mehr als eine, insbesondere im Wesentlichen alle, der Energiemengen (14) zu erzeugen, wobei der gepulste Laserstrahl einen Fokussierwinkel (a) aufweist, der im Bereich zwischen 10 Grad und 18 Grad, optional zwischen 13 Grad und 16 Grad, liegt, der ferner optional im Wesentlichen bei ungefähr 14 Grad liegt; wobei die Energiemengen (14) von einem lichtemittierenden Element (20) über das optische Fasersystem (27), das vorzugsweise eine optische Faser mit einem Kerndurchmesser der optischen Faser aufweist, der im Bereich zwischen 270 µm und 290 µm liegt, insbesondere bei ungefähr 280 µm, zum optischen System (26) geleitet werden;
und/oder
Anwenden der Energiemengen (14) auf die anteriore Stromaschicht (8) im Wesentlichen in vertikaler Richtung;
und/oder
zum Erzeugen zumindest einer oder mehr als einer, insbesondere im Wesentlichen aller, der Energiemengen (14) als mechanische Wellenimpulse als direkte Ursache eines plasmainduzierten Berstimpulses, der zumindest teilweise durch eines oder mehrere erzeugt wird von:
einer direkten Interaktion eines Laserimpulses mit einem Laserziel, das mit dem intraokularen Humor in Fluidverbindung steht;
einer direkten Interaktion eines Laserimpulses mit einem Laserziel, das innerhalb der anterioren Augenkammer (9) platziert ist;
einer direkten Interaktion eines Laserimpulses mit einer Spüllösung innerhalb der anterioren Augenkammer (9); und
einer direkten Interaktion eines Laserimpulses mit der anterioren Stromaschicht (8), insbesondere einer fibrovaskulären Stromastruktur der Stromaschicht (8).

11. Computerlesbares nicht flüchtiges Speichermedium nach zumindest einem der Ansprüche 5 bis 10, das ferner ausführbare Anweisungen umfasst, die, wenn sie von der Steuereinheit (23) ausgeführt werden, das Gerät (18) veranlassen zum:
Verfolgen eines oder von mehr als einem von einer Position, einer Form und einer Bewegung des Auges (1) oder einer der Komponenten des Auges (1) wie z. B. der Iris (4) oder der Pupille (5) in Bezug auf einen Raumreferenzpunkt durch ein Verfolgungsmodul (24), insbesondere ein optisches Verfolgungsmodul (24), und
Anwenden zumindest teilweise der erzeugten vorbestimmten Energiemengen (14), optional jeder der erzeugten vorbestimmten Energiemengen (14), abhängig von dem Verfolgungsergebnis;
und/oder optional ferner umfassend ausführbare Anweisungen, die, wenn sie von der Steuereinheit (23) ausgeführt werden, das Gerät veranlassen zum:
Hemmen des Generatormoduls und/oder Hemmen des Anwendens einer oder mehr als einer Energiemenge (14), wenn das Verfolgungsergebnis eines oder mehrere einer Änderung einer Position, einer Änderung einer Stelle, einer Änderung einer Form und einer Bewegung, und/oder
Neueinstellen der Zieleinstellung der Energiemenge gemäß einem oder mehreren einer Änderung einer Position, einer Änderung einer Stelle, einer Änderung einer Form und einer Bewegung; und/oder
Verbinden eines Augenabschirmungselements (29) mit dem Auge (1), insbesondere der Außenschicht (32) der Hornhaut (10), vor Anwenden einer oder mehr als einer der Energiemengen (14);
wobei das Augenabschirmungselement (29) dazu umgesetzt und positioniert ist, zu verhindern, dass Energiemengen (14) in die posteriore Augenkammer (12) eintreten und/oder auf die Netzhaut (3) des Auges (1) auftreffen;
wobei das Augenabschirmungselement (29) optional in einer Verbundstruktur umgesetzt ist, umfassend eine Absorptionsschicht (30) zum Absorbieren von Energiemengen (14), die auf diese auftreffen, und eine Adhäsionsschicht (31) zur Verbindung mit der äußeren Hornhautschicht (32), insbesondere durch Adhäsionskräfte, wobei die Absorptionsschicht (30) optional eine oder mehr als eine Metallschicht (30), insbesondere Edelstahlschichten, umfasst, und
wobei die Adhäsionsschicht (31) optional eine Schicht (31) offenporigen Materials, insbesondere eine geschäumte Schicht, umfasst, die vorzugsweise ein Polyvinylalkoholmaterial umfasst und/oder daraus hergestellt ist;
wobei das Augenabschirmungselement (29), insbesondere in der Adhäsionsschicht (31), teilweise mit natürlicher Flüssigkeit von der Außenseite der Hornhaut gefüllt ist oder diese absorbiert, wodurch es wie z. B. ein Papiergewebe oder ein Löschpapier an der Hornhaut anhaftet,
wobei das Augenabschirmungselement (29) optional eine konvexe; und/oder kreisförmige Form und/oder einen Durchmesser, der im Bereich zwischen 0,5 mm und 3 mm oder im Bereich zwischen 1 mm und 2 mm oder im Wesentlichen bei ungefähr 1,5 mm liegt, aufweist.

12. Computerlesbares nicht flüchtiges Speichermedium nach zumindest einem der Ansprüche 5 bis 11, das ferner ausführbare Anweisungen umfasst, die, wenn sie von der Steuereinheit (23) ausgeführt werden, das Gerät (18) veranlassen zum:
Abtasten zumindest der Iris (4) oder von Abschnitten dieser und/oder der anterioren Augenkammer (9) zumindest während des Anwendens der Energiemengen (14) durch ein Abtastmodul (24); und Durchführen eines oder mehr als einen der folgenden Schritte:
Bestimmen einer Form der Iris und/oder einer Spur, eines Wegs und/oder einer Abfolge von Zielpunkten, auf die die Energiemengen auftreffen sollen, auf Basis des Abtastergebnisses;
Speichern des Abtastergebnisses nach jeder vorbestimmten Zahl angewandter Energiemengen;
Bestimmen einer tatsächlichen Stelle des Auftreffens oder einer tatsächlichen gemittelten Stelle des Auftreffens auf Basis des Abtastergebnisses, die jeweils eine tatsächliche Stelle auf der anterioren Stromaschicht (8)/der Iris (4) indizieren, an der eine oder mehrere Energiemengen (4) tatsächlich auf der anterioren Stromaschicht (8) auftrafen, und optional Verfolgen der Zielauftreffstellen;
Steuern der Strömung (17) einer Spüllösung innerhalb der anterioren Augenhöhle (9) oder durch diese auf Basis des Abtastergebnisses;
Bestimmen einer Dichte von Pigmenten, insbesondere einer lokalen Dichte von Pigmenten, insbesondere eines Pigmentprofils, auf Basis des Abtastergebnisses, oder wobei zumindest ein Parameter, der für die Dichte, insbesondere die lokale Dichte, von Pigmenten repräsentativ ist, auf Basis des Abtastergebnisses bestimmt werden kann, und Steuern des Erzeugens und/oder Anwendens einer oder mehrerer der Energiemengen (14) auf Basis zumindest teilweise der Dichte von Pigmenten oder des jeweiligen Parameters;
Bestimmen einer Änderung, insbesondere einer lokalen Änderung, der Dichte von Pigmenten oder zumindest eines Parameters, der für die Änderung der Dichte von Pigmenten in der anterioren Stromaschicht (8) repräsentativ ist, und Steuern des Erzeugens und/oder Anwendens der Energiemengen (14) auf Basis der bestimmten Änderung der Dichte von Pigmenten oder des jeweiligen Parameters;
Erzeugen eines oder von mehr als einem Anzeigeobjekt zum Anzeigen auf einem Anzeigebildschirm für einen Benutzer, der das Verfahren durchführt, auf Basis des Abtastergebnisses; und optional Bereitstellen von Betriebsparametern in Zusammenhang mit dem Durchführen des Verfahrens zur Anzeige auf dem Anzeigebildschirm, die insbesondere eines oder mehr als eines umfassen von: einem oder mehr als einem Parameter in Zusammenhang mit den Energiemengen (14), einem oder mehreren Auftreffpunkten (T) einer oder mehrerer angewandter Energiemengen (14) auf der anterioren Stromaschicht (8), insbesondere einem oder mehr als einem von einem oder mehreren vergangenen und künftigen Auftreffpunkten von Energiemengen (14), einer ersten Angabe, die für eine Änderung, insbesondere lokale Änderung, der Dichte von Pigmenten indikativ ist, und einer zweiten Änderung, die für verarbeitete und/oder nicht verarbeitete Oberflächenbereiche der anterioren Oberfläche der Stromaschicht repräsentativ ist.

13. Gerät (18) nach einem der Ansprüche 1 bis 4, dass das computerlesbare nicht flüchtige Speichermedium nach einem der Ansprüche 5 bis 12 umfasst.

14. Steuereinheit (23), die ausführbare Anweisungen umfasst, die, wenn sie auf der Steuereinheit (23) ausgeführt werden, die Steuereinheit (23) dazu veranlassen, das Gerät (18) nach einem der Ansprüche 1 bis 4 dazu zu veranlassen, ein Verfahren zum Ändern der humanen wahrnehmenden Farberscheinung der Iris (4) eines Auges (1) eines Menschen oder eines Tiers durch selektives Verringern der Dichte von Pigmenten (15) der anterioren Stromaschicht (8) der Iris (4) durchzuführen, wobei das Verfahren die Schritte umfasst:
Erzeugen (402) einer Mehrzahl vorbestimmter Energiemengen (14) durch ein Generatormodul (19, 20); und
Anwenden (403) einer oder mehrerer der vorbestimmten Energiemengen (14) durch das Generatormodul (19, 20) auf die anteriore Stromaschicht (8);
wobei jede der vorbestimmten Energiemengen (14) so erzeugt und angewandt wird, dass sie Melanozyten (15) der Stroma (6) zumindest teilweise ablatieren, während Nicht-Melanozytengewebe zumindest der Stroma (6) im Wesentlichen unbeschädigt bleibt;
die vorbestimmten Energiemengen (14) zumindest teilweise so erzeugt und angewandt werden, dass ablatierte Trümmer von Gewebe (16) oder Pigment, wie als unmittelbare Ursache einer oder mehrerer der angewandten Energiemengen (14) erzeugt, in die anteriore Augenkammer (9) ausgeschieden wird, so dass das ausgeschiedene Gewebe (16) durch eine mechanisch erzeugte Strömung (17) einer Spüllösung durch die anteriore Augenkammer (9) oder innerhalb dieser entfernbar ist.

## Revendications

1. Appareil (18) particulièrement adapté et configuré pour changer l'apparence de couleur perceptible humaine de l'iris (4) d'un œil humain ou animal (1) par diminution sélective de la densité de pigments de la couche de stroma antérieure (8) de l'iris (4), l'appareil (18) comprenant :
une source d'énergie (19, 20) comprenant un module de générateur (19) qui est adapté et configuré pour générer une pluralité de quantités d'énergie prédéfinies (14) d'au moins un parmi un type électromagnétique et un type onde mécanique ;
un dispositif de concentration (19, 20) adapté et configuré pour concentrer les quantités d'énergie (14) vers la couche de stroma antérieure (8) de l'iris (4) de l'œil (1) ;
un module de pompage de fluide (21) adapté et configuré pour la génération et l'entretien d'un fluide mécanique prédéfini (17) de solution de rinçage à travers et/ou à l'intérieur de la chambre oculaire antérieure (9) ; et
une unité de commande (23) qui est programmée et configurée pour :
- actionner la source d'énergie (19, 20) et le dispositif de concentration (19, 20) pour appliquer les quantités d'énergie prédéfinies générées (14) à un emplacement prédéfini de la couche antérieure (8) du stroma (6) d'une manière telle qu'un tissu de mélanocyte (15) du stroma (6), qui est impacté par les quantités d'énergie (14), est enlevé par ablation d'une manière telle qu'il est, au moins en partie, évacué dans la chambre oculaire antérieure (9) en tant que cause directe de l'interaction entre les quantités d'énergie (14) et le tissu (15) ; et
- actionner le module de pompage de fluide (21) pour maintenir l'écoulement prédéfini (17) sur une période de temps pendant et/ou directement après l'application des quantités d'énergie (14) au stroma (6), de telle sorte qu'une matière de mélanocyte évacuée (16), au moins en partie, est évacuée fluidiquement de la chambre antérieure (9).

2. Appareil (18) selon la revendication 1, comprenant en outre un ou plus d'un de ce qui suit :
un module de microscope (24), facultativement mis en œuvre en tant que microscope stéréoscopique, disposé et apte à être positionné d'une manière telle qu'un opérateur actionnant l'appareil est en mesure d'observer au moins la surface antérieure de la couche de stroma (8) de l'œil (1), l'appareil (18) comprenant :
un module de lampe à fente (25) comprenant une lampe à fente et des éléments optiques correspondants permettant à l'opérateur/superviseur du procédé de lancer une feuille de lumière dans l'œil (1)/sur la couche de stroma antérieure (8), le module de lampe à fente (25) et le module de microscope (24) étant facultativement adaptés pour une interaction mutuelle mettant ainsi en œuvre un microscope à lampe à fente pour examiner au moins la couche de stroma antérieure (8) de l'iris (4) ;
un élément de protection oculaire (9) mis en œuvre et configuré pour être couplé à l'œil (1), en particulier la couche de cornée externe (32) de façon à empêcher des quantités d'énergie (14) d'entrer dans la chambre oculaire postérieure (12) et/ou d'impacter la rétine (3) de l'œil (1),
l'élément de protection oculaire (29) ayant un ou plus d'un de ce qui suit :
- une structure composite ayant une couche d'absorption (30) pour absorber des quantités d'énergie (14) impactant celle-ci, et une couche d'adhérence (31) pour couplage à la couche de cornée externe (32), en particulier par des forces d'adhésion et/ou de cohésion en particulier pour éviter que l'élément de protection oculaire (9) ne soit amené à flotter à la dérive par du fluide lacrymal, la couche d'absorption comprenant facultativement une ou plusieurs couches métalliques (30), en particulier des couches d'acier inoxydable, et la couche d'adhérence comprenant facultativement une couche de matériau à pores ouverts (31), en particulier une couche en mousse, de préférence comprenant et/ou faite à partir d'un matériau d'alcool polyvinylique ;
- une forme convexe et/ou circulaire et/ou un diamètre se trouvant dans la plage entre 0,5 mm et 3 mm, ou dans la plage entre 1 mm et 2 mm, ou sensiblement à environ 1,5 mm.

3. Appareil (18) selon au moins une des revendications 1 et 2, dans lequel la source d'énergie (19, 20) comprend, en tant que module de générateur, un élément électroluminescent (20), en particulier un dispositif laser, de préférence un dispositif laser déclenché, de préférence un dispositif laser déclenché à double fréquence, de façon davantage préférée un dispositif laser Nd:YAG, le dispositif laser (20) est apte à générer au moins certaines des quantités d'énergie prédéfinies (14) et émettre les quantités d'énergie générées (14) sous la forme d'impulsions lumineuses (14) vers et sur la couche de stroma antérieure (8) de façon à changer la densité de pigments, l'élément électroluminescent (19), en particulier le dispositif laser (19), étant facultativement actionnable ou mis en œuvre pour :
émettre de la lumière, en particulier de la lumière laser puisée, dans une plage de longueurs d'onde entre 488 nm et 580 nm, en particulier 532 nm ;
ou dans une plage de longueurs d'onde entre 976 nm et 1160 nm, ou dans une plage de longueurs d'onde entre 1044 et 1082 nm, ou avec une longueur d'onde correspondant sensiblement à 1064 nm ;
générer des impulsions lumineuses (14) ayant une durée dans la plage entre 2 ns et 6 ns, en particulier 4 ns ;
générer des impulsions lumineuses (14) ayant une énergie d'environ 2 mJ ;
générer des impulsions lumineuses (14) avec une fréquence d'impulsions dans la plage entre 3 Hz et 300 Hz ;
générer des impulsions lumineuses (14) ayant une puissance de crête comprise entre 0,1 MW et 0,5 MW ;
générer un point laser ayant un diamètre compris entre 200 µm et 600 µm, de préférence dans la plage d'environ 400 µm ;
la source d'énergie (19, 20) comprenant facultativement :
- un système optique (26) mis en œuvre et configuré pour générer un faisceau laser pulsé ayant un angle de concentration (a) compris dans la plage entre 10 degrés et 18 degrés, facultativement entre 13 degrés et 16 degrés, en outre facultativement s'étendant sensiblement à environ 14 degrés, le système optique (26) étant facultativement mis en œuvre et configuré pour appliquer les quantités d'énergie (14) à la couche de stroma antérieure (8) sensiblement dans une direction verticale ;
- un système de fibre optique (27) mis en œuvre et configuré pour guider des quantités d'énergie (14) générées par un élément électroluminescent (20) vers l'emplacement cible (T), le système de fibre optique (27) comprenant facultativement une fibre optique ayant un diamètre de noyau de fibre optique compris dans la plage entre 270 µm et 290 µm, en particulier à environ 280 µm, le système de fibre optique (27) étant facultativement couplé entre l'élément électroluminescent (20) et le système optique (26) pour guider des quantités d'énergie (14) de l'élément électroluminescent (20), situé à un emplacement éloigné, au système optique (26).

4. Appareil (18) selon la revendication 1 ou 2, dans lequel la source d'énergie comprend, en tant que module de générateur,
un générateur d'onde de pression, le générateur d'onde de pression étant actionnable pour générer au moins certaines des quantités d'énergie prédéfinies et émettre les quantités d'énergie générées sous la forme d'ondes de pression mécaniques, en particulier des ondes de choc ou de souffle, vers et sur la couche de stroma antérieure (8) de façon à changer la densité de pigments, le générateur d'onde de pression étant facultativement actionnable ou mis en œuvre pour :
générer les ondes de pression par cavitation induite à l'intérieur d'un liquide qui est en contact liquide avec le liquide oculaire contenu dans la chambre oculaire antérieure (9) ;
la cavitation induite étant facultativement générée en impactant un objet cible avec des ondes électromagnétiques, en particulier de la lumière laser, de préférence de la lumière laser puisée, et/ou par une source d'ultrasons, en particulier un générateur piézoélectrique.

5. Support de stockage non-transitoire lisible par ordinateur, comprenant des instructions exécutables qui, une fois exécutées sur une unité d'ordinateur ou de contrôleur (23), amène l'unité d'ordinateur ou de contrôleur (23) à amener l'appareil (18) selon l'une quelconque des revendications 1 à 4 à mettre en œuvre un procédé pour changer l'apparence de couleur perceptible humaine de l'iris (4) d'un œil humain ou animal (1) par diminution sélective de la densité de pigments (15) de la couche de stroma antérieure (8) de l'iris (4), le procédé comprenant les étapes :
- générer (402), par un module de générateur (19, 20), une pluralité de quantités d'énergie prédéfinies (14) ; et
- appliquer (403), par le module de générateur (19, 20), une ou plusieurs parmi les quantités d'énergie prédéfinies (14) à la couche de stroma antérieure (8) ;
- chacune des quantités d'énergie prédéfinies (14) étant générée et appliquée de telle sorte qu'elles enlèvent par ablation, au moins en partie, des mélanocytes (15) du stroma (6) tout en laissant essentiellement non endommagé un tissu exempt de mélanocytes d'au moins le stroma (6) ;
- les quantités d'énergie prédéfinies (14) étant au moins en partie générées et appliquées d'une manière telle que des débris de pigment ou de tissu enlevé par ablation (16), qui sont générés en tant que cause immédiate d'une ou plusieurs des quantités d'énergie prédéfinies (14), sont évacués dans la couche oculaire antérieure (9), de telle sorte que le tissu évacué (16) peut être retiré par un écoulement généré mécaniquement (17) de solution de rinçage à travers ou à l'intérieur de la chambre oculaire antérieure (9).

6. Support de stockage non-transitoire lisible par ordinateur selon la revendication 5, comprenant des instructions exécutables supplémentaires qui, une fois exécutées par l'unité de contrôleur (23), amènent l'appareil (18) à :
fournir un écoulement généré mécaniquement (17) de solution de rinçage à travers ou à l'intérieur de la chambre oculaire antérieure (9) ; et
retirer les débris de pigment/tissu (16) de l'œil (1) au moyen de l'écoulement généré (17).

7. Support de stockage non-transitoire lisible par ordinateur selon la revendication 5 ou 6, comprenant des instructions exécutables supplémentaires qui, une fois exécutées par l'unité de contrôleur (23), amènent l'appareil (18) à mettre en œuvre une ou plus d'une parmi les étapes suivantes :
- maintenir l'écoulement généré mécaniquement (17) sur un laps de temps respectivement prédéterminé au moins pendant et/ou après application de la quantité d'énergie prédéfinie (14) à la couche de stroma antérieure (8) ;
- maintenir l'écoulement généré mécaniquement (17) également pendant un laps de temps prédéfini avant application de la quantité d'énergie prédéfinie (14) à la couche de stroma antérieure (8) ;
- maintenir l'écoulement généré mécaniquement (17) sur au moins un laps de temps prédéterminé selon un profil de débit prédéterminé respectif, le profil de débit prédéterminé étant de préférence constant dans le temps, au moins sur un laps de temps et facultativement sur chaque laps de temps, au moins un parmi un point de début et un point de fin d'au moins un laps de temps étant facultativement déclenché par la génération et/ou l'application de la quantité d'énergie prédéfinie (14) ;
- l'écoulement généré mécaniquement (17) comprenant facultativement, au moins pendant une première période de temps prédéterminée, un écoulement laminaire et/ou, au moins pendant une seconde période de temps prédéterminée, un écoulement turbulent.

8. Support de stockage non-transitoire lisible par ordinateur selon au moins une des revendications 5 à 7, comprenant des instructions exécutables supplémentaires qui, une fois exécutées par l'unité de contrôleur (23), amènent l'appareil (18) à mettre en œuvre les étapes suivantes :
- séparer, de préférence par un module de séparation (24), de façon davantage préférée sur la base d'une image capturée de l'iris (4), au moins une partie de la surface de la couche de stroma antérieure (8) en un nombre de sections de surface prédéfinies, ayant de préférence une taille prédéterminée, et
- appliquer un nombre respectif de quantités d'énergie prédéfinies (14) à une ou plusieurs sections de surface ;
les sections de surface prédéfinies étant facultativement traitées selon une succession prédéfinie de sections de surface, la succession prédéfinie étant de préférence déterminée par le module de séparation (24) ;
les sections de surface prédéterminées, en particulier la taille d'une ou plusieurs des sections de surface prédéterminées, et/ou la succession particulière de sections de surface à l'intérieur de la séquence de traitement et/ou la teneur en énergie/puissance de la quantité d'énergie étant facultativement déterminées sur la base de la densité de pigments, et/ou de l'emplacement spécifique de la surface sur l'iris (4) et/ou de la taille globale de l'iris (4) ; au moins un paramètre de l'écoulement généré mécaniquement (17) étant facultativement déterminé sur la base d'un ou plus d'un parmi :
- l'emplacement spécifique d'une section de surface respectivement traitée,
- la succession particulière des sections de surface,
- la densité de pigments,
- la taille d'une section de surface respective,
- un ou plus d'un paramètre lié à la génération et/ou l'application des quantités d'énergie (14).

9. Support de stockage non-transitoire lisible par ordinateur selon au moins une des revendications 5 à 8, comprenant des instructions exécutables supplémentaires qui, une fois exécutées par l'unité de contrôleur (23), amènent l'appareil (18) à mettre en œuvre les étapes suivantes, dans lesquelles : au moins une ou plus d'une des quantités d'énergie prédéfinies (14) sont générées et appliquées à la couche de stroma (8) sous la forme d'au moins une parmi :
- une ou plusieurs ondes électromagnétiques (14), en particulier des impulsions d'ondes électromagnétiques (14), les ondes électromagnétiques étant de préférence générées par un dispositif laser (19, 20), en particulier un dispositif laser pulsé (19, 20), plus particulièrement un dispositif laser déclenché (19, 20), en particulier un dispositif laser déclenché à double fréquence, et
- une ou plusieurs ondes de pression mécaniques, en particulier des ondes de choc ou de souffle, de préférence induites par cavitation, en particulier une cavitation induite par plasma, plus particulièrement directement induites par un souffle de plasma et/ou induites par une impulsion d'onde électromagnétique, les ondes de pression mécaniques étant générées à l'intérieur d'un liquide contenu dans ou en contact de fluide direct avec la chambre oculaire antérieure (9),
et/ou comprenant des instructions supplémentaires qui, une fois exécutées par l'unité de contrôleur (23), amènent l'appareil (18) à :
déterminer la densité de pigment locale et/ou l'épaisseur de couche de mélanocytes au niveau de la couche de stroma antérieure (8), la génération et/ou l'application locale d'une ou plusieurs des quantités d'énergie (14) étant mises en œuvre en fonction d'une densité locale respective de pigments et/ou d'une épaisseur locale de la couche de mélanocytes de la couche de stroma antérieure (8).

10. Support de stockage non-transitoire lisible par ordinateur selon au moins une des revendications 5 à 9, comprenant des instructions exécutables supplémentaires qui, une fois exécutées par l'unité de contrôleur (23), amènent l'appareil (18) à générer au moins une ou plus d'une, en particulier sensiblement la totalité, des quantités d'énergie (14) sous la forme d'impulsions d'ondes électromagnétiques (14), en particulier pour une application directe à un tissu de l'iris (4), par actionnement d'un dispositif laser (19, 20) pour :
- générer au moins une partie des quantités d'énergie (14) pour avoir une plage de longueurs d'onde entre 488 et 580 nm, ou dans une plage de longueurs d'onde entre 522 nm et 541 nm, ou avec une longueur d'onde correspondant sensiblement à 532 nm, ou dans une plage de longueurs d'onde entre 976 nm et 1160 nm, ou dans une plage de longueurs d'onde entre 1044 et 1082 nm, ou avec une longueur d'onde correspondant sensiblement à 1064 nm ; et/ou
- générer au moins une partie des quantités d'énergie (14) avec une fréquence d'impulsions dans la plage entre 3 Hz et 300 Hz ; et/ou
- générer au moins une partie des quantités d'énergie (14) pour avoir une longueur d'impulsions comprise dans la plage entre 2 ns et 6 ns, ou correspondant sensiblement à 4 ns ; et/ou
- générer, par actionnement ou ajustement d'un système optique (26) du dispositif laser (19, 20), un faisceau laser pulsé pour générer au moins une ou plus d'une, en particulier sensiblement la totalité, des quantités d'énergie (14), le faisceau laser pulsé ayant un angle de concentration (a) compris dans la plage entre 10 degrés et 18 degrés, facultativement entre 13 degrés et 16 degrés et, en outre, facultativement s'étendant sensiblement à environ 14 degrés ; les quantités d'énergie (14) étant guidées d'un élément électroluminescent (20) au système optique (26) par l'intermédiaire d'un système à fibre optique (27), ayant de préférence une fibre optique avec un diamètre de noyau de fibre optique compris dans la plage entre 270 µm et 290 µm, en particulier à environ 280 µm ; et/ou
- appliquer les quantités d'énergie (14) à la couche de stroma antérieure (8) sensiblement dans une direction verticale ; et/ou
générer au moins une ou plus d'une, en particulier sensiblement la totalité, des quantités d'énergie (14) sous la forme d'impulsions d'onde mécanique en tant que cause directe d'une impulsion en rafale induite par plasma générée au moins en partie par une ou plus d'une parmi :
- une interaction directe d'une impulsion laser avec une cible laser qui est en communication fluidique avec l'humeur intraoculaire ;
- une interaction directe d'une impulsion laser avec une cible laser qui est placée à l'intérieur de la chambre oculaire antérieure (9) ;
- une interaction directe d'une impulsion laser avec une solution de rinçage à l'intérieur de la chambre oculaire antérieure (9) ; et/ou
- une interaction directe d'une impulsion laser avec la couche de stroma antérieure (8), en particulier une structure de stroma fibrovasculaire de la couche de stroma (8).

11. Support de stockage non-transitoire lisible par ordinateur selon au moins une des revendications 5 à 10, comprenant des instructions exécutables supplémentaires qui, une fois exécutées par l'unité de contrôleur (23), amènent l'appareil (18) à :
suivre, par un module de suivi (24), en particulier un module de suivi optique (24), un ou plus d'un parmi une position, une forme et un mouvement de l'œil (1) ou un des composants de l'œil (1), tels que l'iris (4) ou la pupille (5), par rapport à un point de référence spatial, et
appliquer, au moins en partie, les quantités d'énergie prédéfinies générées (14), facultativement chacune des quantités d'énergie prédéfinies générées (14), en fonction du résultat de suivi ;
et facultativement comprenant en outre des instructions exécutables qui, une fois exécutées par l'unité de contrôleur (23), amènent l'appareil à :
- bloquer le module de générateur et/ou empêcher l'application d'une ou plus d'une quantité d'énergie (14) dans le cas où le résultat de suivi indique un ou plusieurs parmi un changement de position, un changement d'emplacement, un changement de forme et un mouvement, et/ou
- redéfinir le réglage cible de la quantité d'énergie en fonction d'un ou plusieurs parmi un changement de position, un changement d'emplacement, un changement de forme et un mouvement ; et/ou
- coupler, avant d'appliquer une ou plus d'une des quantités d'énergie (14), un élément de protection oculaire (29) à l'œil (1), en particulier la couche externe (32) de la cornée (10) ;
l'élément de protection oculaire (29) étant mis en œuvre et positionné pour empêcher des quantités d'énergie (14) d'entrer dans la chambre oculaire postérieure (12) et/ou d'impacter la rétine (3) de l'œil (1) ;
l'élément de protection oculaire (29) étant facultativement mis en œuvre dans une structure composite comprenant une couche d'absorption (30) pour absorber des quantités d'énergie (14) impactant celle-ci, et une couche d'adhérence (31) pour couplage à la couche de cornée externe (32), en particulier par des forces d'adhésion, la couche d'absorption (30) comprenant facultativement une ou plus d'une couche métallique (30), en particulier des couches d'acier inoxydable, et la couche d'adhérence (31) comprenant facultativement une couche de matériau à pores ouverts (31), en particulier une couche en mousse, de préférence comprenant et/ou faite à partir d'un matériau d'alcool polyvinylique ;
l'élément de protection oculaire (29) étant partiellement, en particulier dans la couche d'adhérence (31), rempli de ou absorbant du liquide naturel provenant du côté externe de la cornée, adhérant ainsi à la cornée, comme le ferait un mouchoir en papier ou un buvard ;
l'élément de protection oculaire (29) ayant facultativement une forme convexe et/ou circulaire et/ou un diamètre se trouvant dans la plage entre 0,5 mm et 3 mm, ou dans la plage entre 1 mm et 2 mm, ou sensiblement à environ 1,5 mm.

12. Support de stockage non-transitoire lisible par ordinateur selon au moins une des revendications 5 à 11, comprenant des instructions exécutables supplémentaires qui, une fois exécutées par l'unité de contrôleur (23), amènent l'appareil (18) à :
balayer, par un module de balayage (24), au moins l'iris (4) ou des sections de celui-ci, et/ou la chambre oculaire antérieure (9) au moins pendant l'application des quantités d'énergie (14) ; et réaliser une ou plus d'une des étapes suivantes :
- déterminer une forme de l'iris et/ou une trajectoire, un trajet et/ou une succession de points cibles à impacter avec les quantités d'énergie sur la base du résultat de balayage ;
- stocker le résultat de balayage après chaque nombre prédéterminé de quantités d'énergie appliquées ;
- déterminer, sur la base du résultat de balayage, un emplacement réel d'impact ou un emplacement moyen réel d'impact indiquant respectivement un emplacement réel sur la couche de stroma antérieure (8)/l'iris (4) où une ou plusieurs quantités d'énergie (4) ont en fait impacté la couche de stroma antérieure (8) et, facultativement, suivent les emplacements cibles d'impact ;
- réguler, sur la base du résultat de balayage, l'écoulement (17) de solution de rinçage à l'intérieur de ou à travers la cavité oculaire antérieure (9) ;
- sur la base du résultat de balayage, déterminer une densité de pigments, en particulier une densité locale de pigments, en particulier un profil de pigment, ou au moins un paramètre représentant la densité, en particulier la densité locale, de pigments, pouvant être déterminé sur la base du résultat de balayage, et commander la génération et/ou l'application d'une ou plus d'une des quantités d'énergie (14) sur la base, au moins en partie, de la densité de pigments ou du paramètre respectif ;
- sur la base du résultat de balayage, déterminer un changement, en particulier un changement local, de densité de pigments, ou au moins un paramètre représentant le changement de densité de pigments dans la couche de stroma antérieure (8), et commander la génération et/ou l'application des quantités d'énergie (14) sur la base du changement déterminé de la densité de pigments ou du paramètre respectif ;
- générer, sur la base du résultat de balayage, un ou plus d'un objet d'affichage à afficher sur un écran d'affichage à un opérateur exécutant le procédé ; et, facultativement, fournir, pour un affichage sur l'écran d'affichage, des paramètres de fonctionnement liés à l'exécution du procédé, en particulier comprenant un ou plus d'un parmi : un ou plus d'un paramètre lié aux quantités d'énergie (14), un ou plusieurs points d'impact (T) d'une ou plusieurs quantités d'énergie appliquées (14) sur la couche de stroma antérieure (8), en particulier un ou plus d'un parmi un ou plusieurs points d'impact passés et futurs de quantités d'énergie (14), une première indication représentant un changement, en particulier un changement local, de la densité de pigments, et une seconde indication représentant des régions de surface traitées et/ou non traitées de la surface antérieure de la couche de stroma.

13. Appareil (18) selon l'une quelconque des revendications 1 à 4, comprenant le support de stockage non-transitoire lisible par ordinateur selon l'une quelconque des revendications 5 à 12.

14. Unité de contrôleur (23) comprenant des instructions exécutables qui, une fois exécutées sur l'unité de contrôleur (23), amènent l'unité de contrôleur (23) à amener l'appareil (18) selon l'une quelconque des revendications 1 à 4 à mettre en œuvre un procédé pour changer l'apparence de couleur perceptible humaine de l'iris (4) d'un œil humain ou animal (1) par diminution sélective de la densité de pigments (15) de la couche de stroma antérieure (8) de l'iris (4), le procédé comprenant les étapes :
- générer (402), par un module de générateur (19, 20), une pluralité de quantités d'énergie prédéfinies (14) ; et
- appliquer (403), par le module de générateur (19, 20), une ou plusieurs des quantités d'énergie prédéfinies (14) à la couche de stroma antérieure (8) ;
- chacune des quantités d'énergie prédéfinies (14) étant générée et appliquée de telle sorte qu'elles enlèvent par ablation, au moins en partie, des mélanocytes (15) du stroma (6) tout en laissant essentiellement non endommagé un tissu exempt de mélanocytes du stroma (6) ;
- les quantités d'énergie prédéfinies (14) au moins en partie générées et appliquées d'une manière telle que des débris de pigment ou de tissu enlevé par ablation (16), qui sont générés en tant que cause immédiate d'une ou plus d'une des quantités d'énergie appliquées (14), sont évacués dans la chambre oculaire antérieure (9), de telle sorte que le tissu évacué (16) peut être retiré par un écoulement généré mécaniquement (17) de solution de rinçage à travers ou à l'intérieur de la chambre oculaire antérieure (9).
